(19) Europäisches Patentamt
European Patent Office
Office européen des brevets

(11) Veröffentlichungsnummer: **0 305 330 A1**

# (12) EUROPÄISCHE PATENTANMELDUNG

(21) Anmeldenummer: **88810559.0**

(22) Anmeldetag: **16.08.88**

(51) Int. Cl.[4]: **C 07 D 233/90**
C 07 D 405/10, A 01 N 43/50

(30) Priorität: **25.08.87 CH 3242/87**

(43) Veröffentlichungstag der Anmeldung:
**01.03.89 Patentblatt 89/09**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

**JANSSEN PHARMACEUTICA N.V.**
**Turnhoutsebaan 30**
**B-2340 Beerse (BE)**

(72) Erfinder: **Szczepanski, Henry, Dr.**
**Bodenmatt**
**CH-4323 Wallbach (CH)**

**Wals, Lourens, Dr.**
**Albert Van Dijckstraat 9**
**B-2300 Turnhout (BE)**

(54) **Imidazolderivate.**

(57) 1,5-substituierte Imidazolderivate der Formel I

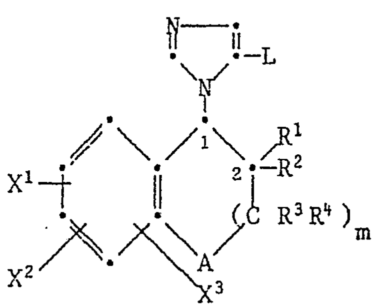

(I),

die stereochemisch isomeren Formen davon, sowie deren Salze, haben nützliche herbizide Eigenschaften. Die Substituenten $R^1$, $R^2$, $R^3$, $R^4$, $X^1$, $X^2$, $X^3$, A, L und m haben die hierin definierten Bedeutungen.

EP 0 305 330 A1

## Beschreibung

### Imidazolderivate

Die vorliegende Erfindung betrifft neue herbizid wirksame 1,5-substituierte Imidazolderivate, diese Wirkstoffe enthaltende agrochemische Mittel und ein Verfahren zur Bkämpfung von Unkräutern, vorzugsweise zur selektiven Bekämpfung von Unkräutern in Nutzpflanzenkulturen. Ferner betrifft die Erfindung auch Verfahren zur Herstellung der neuen Verbindungen.

Die herbizid wirksamen 1,5-substituierte Imidazolderivate entsprechen der Formel I

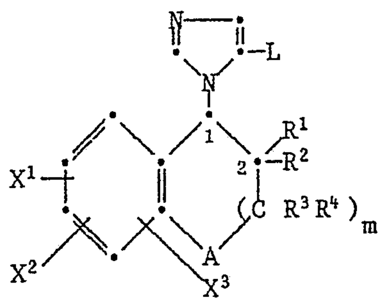 (I),

den stereochemisch isomeren Formen davon, sowie deren Salzen, worin m die Zahl null oder eins,
A eine Gruppe

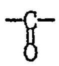 , 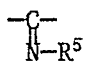 , 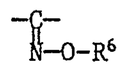 ,

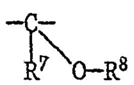 , 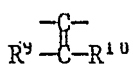 , 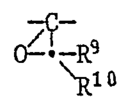 ,

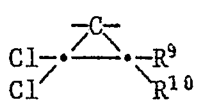 , 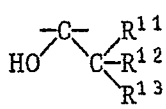 oder 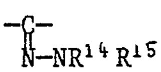

oder falls m für die Zahl null steht auch eine Gruppe

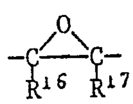 ,

L Cyano oder eine Gruppe

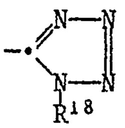 , 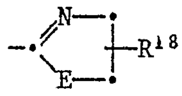 , 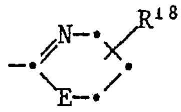 , 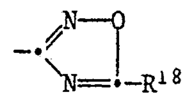 ,

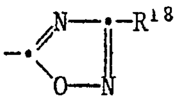 , 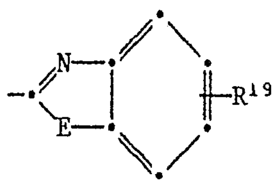 , 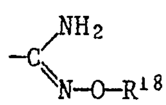

oder 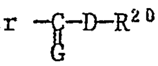 ,

$X^1$ Wasserstoff, $C_1$-$C_5$-Alkyl, $C_1$-$C_5$-Alkoxy, Halogen, $C_2$-$C_5$-Alkenyl, Nitro, Amino, $C_1$-$C_5$-Alkylcarbonylamino, Trifluormethyl oder Difluormethoxy,
$x^2$ Wasserstoff, $C_1$-$C_5$-Alkyl, Halogen oder $C_1$-$C_5$-Alkoxy,
$X^3$ Wasserstoff oder Halogen,
$R^1$ und $R^2$ unabhängig voneinander Wasserstoff, $C_1$-$C_5$-Alkyl oder $C_2$-$C_5$-Alkenyl, oder
$R^1$ und $R^2$ zusammen mit dem sie tragenden Kohlenstoffatom einen spirocyclischen $C_3$-$C_7$-Cycloalkanring,
$R^3$ Wasserstoff, $C_1$-$C_5$-Alkyl, Halogen, $C_1$-$C_5$-Alkoxy oder Hydroxy,
$R^4$ Wasserstoff, $C_1$-$C_5$-Alkyl, Halogen oder $C_1$-$C_5$-Alkoxy, oder
$R^3$ und $R^4$ zusammen mit dem sie tragenden Kohlenstoffatom eine Carbonylgruppe, oder
$R^2$ und $R^3$ eine $C_2$-$C_5$-Alkylenbrücke bedeuten, wobei
$R^5$, $R^6$, $R^7$, $R^9$ und $R^{16}$ jeweils für Wasserstoff, $C_1$-$C_5$-Alkyl, -$CH_2$-$C_2$-$C_5$-Alkenyl, Benzyl oder Phenyl,
$R^8$ für Wasserstoff, -CO-$R^{21}$ oder -$SO_2$-$R^{22}$
$R^{10}$, $R^{11}$, $R^{12}$ und $R^{17}$ jeweils unabhängig voneinander für Wasserstoff, $C_1$-$C_5$-Alkyl, $C_2$-$C_5$-Alkenyl oder Phenyl
$R^{13}$ für Wasserstoff, Halogen, $C_1$-$C_5$-Alkoxy, Amino, $C_1$-$C_5$-Alkylamino oder Di-$C_1$-$C_5$-Alkylamino,
$R^{14}$ und $R^{15}$ unabhängig voneinander für Wasserstoff oder $C_1$-$C_5$-Alkyl,
E für Sauerstoff, Schwefel oder -$NR^{18}$-,
$R^{18}$ für Wasserstoff oder $C_1$-$C_5$-Alkyl,
$R^{19}$ für Wasserstoff, $C_1$-$C_5$-Alkyl, $C_1$-$C_5$-Alkoxy, Halogen, Nitro, Trifluormethyl oder Difluormethoxy,
G für Sauerstoff, Schwefel oder =N-$R^{20}$,
D für Sauerstoff, Schwefel, -$NR^{23}$-,
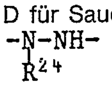 oder 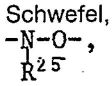
$R^{20}$, $R^{23}$, $R^{24}$ und $R^{25}$ unabhängig voneinander für Wasserstoff, $C_1$-$C_5$-Alkyl, -$CH_2$-$C_2$-$C_5$-Alkenyl, -$CH_2$-$C_2$-$C_6$-Alkinyl, $C_3$-$C_7$-Cycloalkyl oder mit $C_3$-$C_7$-Cycloalkyl, $C_3$-$C_7$-Cycloalkoxy, Phenyl, $C_1$-$C_5$-Alkoxy, Hydroxy, Cyan,

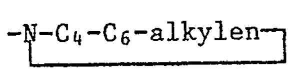 ,

Carboxyl oder $C_1$-$C_5$-Alkoxycarbonyl substituiertes $C_1$-$C_5$-Alkyl, oder
$R^{20}$ und $R^{23}$ zusammen mit dem sie tragenden Stickstoffatom für einen gegebenenfalls durch $C_1$-$C_5$-Alkyl substituierten Piperidinyl-, Pyrrolidinyl-, Morpholinyl- oder Thiomorpholinylring,
$R^{21}$ für gegebenenfalls durch $C_1$-$C_5$-Alkoxy oder 1 bis 3 Halogenatome substituiertes $C_1$-$C_5$-Alkyl oder für gegebenenfalls durch 1 bis 2 Substituenten aus der Gruppe $C_1$-$C_5$-Alkyl, $C_1$-$C_5$-Alkoxy, Halogen, $C_1$-$C_5$-Halogenalkyl, $C_1$-$C_5$-Halogenalkoxy, Nitro oder Benzyl substituiertes Phenyl, und
$R^{22}$ für $C_1$-$C_5$-Alkyl, $C_1$-$C_5$-Halogenalkyl oder gegebenenfalls durch 1 bis 2 Substituenten aus der Gruppe $C_1$-$C_5$-Alkyl, $C_1$-$C_5$-Alkoxy, $C_1$-$C_5$-Halogenalkyl, $C_1$-$C_5$-Halogenalkoxy, Halogen oder Nitro substituiertes Phenyl stehen.

Imidazolcarbonsäureester mit teilhydrierten kondensierten polycyclischen Substituenten sind aus der Europäischen Patentanmeldung EP-A-207563 mit ihren biologischen Wirkungen und ihren Herstellungsmethoden bekannt.

Ueberraschenderweise besitzen die erfindungsgemässen Verbindungen der Formel I starke herbizide Eigenschaften, welche es erlauben, Unkräuter zu bekämpfen. Diese Eigenschaft gewinnt dadurch an Bedeutung, dass einige Nutzpflanzenkulturen nicht durch die Behandlung mit Verbindungen der Formel I geschädigt werden oder, dass nur bei sehr hohen Dosierungen geringfügige Schädigungen der Kultur

auftreten. Somit sind die Verbindungen der Formel I wertvolle Selektivherbizide in Nutzpflanzenkulturen wie Getreide, Zuckerrüben, Raps, Sojabohnen, Reis und Mais. Besonders in Reiskulturen kann ein breiter Bereich von Aufwandmengen angewendet werden, insbesondere dann, wenn es sich bei den Reiskulturen um verpflanzten Reis handelt und wenn die Verbindungen der Formel I nach der Verpflanzung ausgebracht werden.

Die aktiven Wirkstoffe der Formel I werden üblicherweise bei Aufwandmengen zwischen 0,01 und 5,0 kg Aktivsubstanz pro Hektar angewendet um zufriedenstellende Resultate zu erzeugen. Wenn die Umweltbedingungen es erfordern, können die Aufwandmengen in geeigneten Fällen die oben angegebenen Grenzwerte überschreiten. Die bevorzugten Aufwandmengen liegen jedoch im allgemeinen zwischen 0,02 kg und 1,0 kg Wirkstoff pro Hektar.

In den Definitionen ist unter Alkyl geradkettiges oder verzweigtes Alkyl zu verstehen; z.B. Methyl, Aethyl, n-Propyl, i-Propyl oder die vier isomeren Butyl, oder die Isomeren, welche unter der limitierenden Anzahl von bis zu 5 Kohlenstoffatomen konstruiert werden können. Unter Alkoxy ist zu verstehen: Methoxy, Aethoxy, Propyloxy, die vier isomeren Butyloxy oder die Pentyloxyisomeren, insbesondere aber Methoxy, Aethoxy oder Isopropyloxy.

Halogen bedeutet in den obigen Definitionen Fluor, Chlor, Brom oder Jod, worunter Fluor und Chlor bevorzugt sind. Alkenyl bedeutet beispielsweise Vinyl, 1-Propenyl, 1-Methylpropenyl, 1-Butenyl, Allyl, Vinyl, 1-Propenyl, 1-Methylpropenyl, 1-Butenyl, 3-Butenyl, 2-Butenyl, 2-Pentenyl, 3-Pentenyl, 1-Pentenyl, Methallyl oder 3-Methyl-2-butenyl, wobei Allyl und Methallyl bevorzugt sind. Beispiele für Alkinyl sind Aethinyl, Propargyl, 1-Propinyl, 1-Butinyl, 2-Butinyl, 3-Butinyl, 2-Pentinyl, 3-Pentinyl oder 4-Pentinyl, vorzugsweise aber Propargyl. Cycloalkyl bedeutet im allgemeinen Cyclopropyl, Cyclobutyl, Cyclopentyl, Cyclohexyl oder Cycloheptyl, vorzugsweise Cyclopentyl oder Cyclohexyl.

Die unter A definierten Ringglieder entsprechen im wesentlichen den aus der Carbonylfunktion ableitbaren Strukturelementen wie der Iminfunktion, der Oximfunktion, der Hydrazonfunktion, der exocyclischen Alkylidenfunktion, der Oxiranfunktion, der Dichlorcyclopropanfunktion und der Hydroxyfunktion sowie den daraus wiederum ableitbaren Dehydrat- und Oxiranfunktionen.

Für den Fall, dass $R^2$ und $R^3$ gemeinsam eine Alkylenbrücke bilden, entstehen aus diesen beiden Substituenten annellierte, gesättigte Kohlenwasserstoffringe, welche mit dem gesättigten, das Ringglied A enthaltenden Ring eine gemeinsame Bindung haben.

Die unter die Definition von L fallenden Gruppen sind Carbonsäure-Derivate wie Carbonsäureester, Carbonsäurenitrile, Carbonsäureamide, Carbonimidsäuren, Carbonsäurehydrazide, Amidoxime, Hydroxamsäuren, Amidine sowie die diversen cyclisierten Abkömmlinge dieser Funktionen wie Tetraline, Oxazoline, Oxazine, Oxadiazole, oder Benzoxazole sowie die entsprechenden Derivate der Thiocarbonsäuren oder Dithiocarbonsäuren.

Die Erfindung umfasst ebenfalls die Salze, die die Verbindungen der Formel I mit organischen oder anorganischen Basen wie Aminen, Alkalimetallbasen und Erdalkalimetallbasen oder quaternären Ammoniumbasen oder mit organischen oder anorganischen Säuren wie Mineralsäuren, Sulfonsäuren, Carbonsäuren oder phosphorhaltigen Säuren bilden können.

Beispiele salzbildender Mineralsäuren sind Fluorwasserstoffsäure, Chlorwasserstoffsäure, Bromwasserstoffsäure, Jodwasserstoffsäure, Schwefelsäure, Salpetersäure, Chlorsäure, Perchlorsäure oder Phosphorsäure. Bevorzugte salzbildende Sulfonsäuren sind Toluolsulfonsäuren, Benzosulfonsäure, Methansulfonsäure oder Trifluormethansulfonsäure. Als salzbildende Carbonsäuren seien vorzugsweise Essigsäure, Trifluoressig säure, Benzoesäure, Chloressigsäure, Phthalsäure, Maleinsäure, Malonsäure und Zitronensäure genannt. Phosphor-haltige Säuren sind die verschiedenen Phosphonsäuren, phosphonigen Säuren and Phosphinsäuren.

Bevorzugte salzbildende Alkalimetallhydroxide und Erdalkalimetallhydroxide sind die Hydroxide von Lithium, Natrium, Kalium, Magnesium oder Calcium, insbesondere aber die von Natrium oder Kalium. Beispiele von geeigneten salzbildenden Aminen sind primäre, sekundäre und tertiäre aliphatische und aromatische Amine wie Methylamin, Aethylamin, Propylamin, Isopropylamin, die vier Butylaminisomeren, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und Isochinolin. Bevorzugte Amine sind Aethylamin, Propylamin, Diäthylamin ode Triäthylamin, insbesondere aber Isopropylamin, Diäthanolamin und 1,4-Diazabicyclo[2.2.2]octan. Beispiele für quaternäre Ammoniumbasen sind im allgemeinen die Kationen von Halogenammoniumsalzen, beispielsweise das Tetramethylammoniumkation, das Trimethylbenzylammoniumkation, das Tetraäthylammoniumkation, das Trimthyläthylammoniumkation und auch das Ammoniumkation.

Die Erfindung umfasst alle optischen Isomeren der Verbindungen der Formel I, sowie gegebenenfalls auftretende Diastereomere. Diastereomere können in den Isomerengemischen vorhanden sein, wenn die Verbindungen der Formel I mehr als ein asymmetrisch substituiertes Kohlenstoffatom enthalten. Sofern keine nähere Angabe gemacht wird, umfasst die chemische Bezeichnung von Verbindungen Gemische von stereochemisch isomeren Formen. Die Mischungen enthalten unter Umständen alle Diastereomeren und Enatiomeren der zugrundeliegenden Molekülstruktur.

Die reinen isomeren Formen dieser optisch aktiven Verbindungen können aus den Mischungen durch übliche Trennungsmethoden erhalten werden. Wird im Einzelfall eine spezifische stereochemische Form gewünscht, so wird diese Verbindung vorzugsweise durch stereoselektive Syntheseverfahren hergestellt. In diesen Verfahren kommen mit Vorteil reine Formen der optisch aktiven Ausgangsmaterialien zur Anwendung.

Unter den Verbindungen der Formel I sind diejenigen bevorzugt, in denen entweder

a) $X^2$ und $X^3$ Wasserstoff bedeuten, oder

b) $X^1$ Wasserstoff, Chlor, Methyl oder Methoxy bedeutet, oder

c) L für $-COOR^{20}$ oder $-CONR^{20}R^{23}$ steht, oder

d) $R^1$, $R^2$, $R^3$ und $R^4$ unabhängig voneinander für Wasserstoff oder Methyl stehen, oder

e) A für die Gruppe

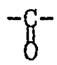 , $-CHOH-$ , 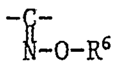 , 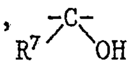 , 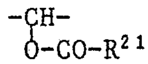 , 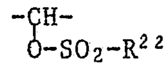 oder

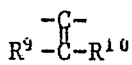

steht, worin $R^6$, $R^7$, $R^9$ und $R^{10}$ unabhängig voneinander jeweils für Wasserstoff, $C_1$-$C_5$-Alkyl oder Phenyl, $R^{21}$ für $C_1$-$C_5$-Alkyl, $C_1$-$C_5$-Chloralkyl, $C_2$-$C_4$-Alkoxyalkyl oder Phenyl und $R^{22}$ für $C_1$-$C_5$-Alkyl oder gegebenenfalls durch Methyl, Chlor oder Nitro substituiertes Phenyl stehen.

Unter den Verbindungen der Untergruppe c) sind diejenigen besonders bevorzugt in denen L für Carboxyl, Carbamoyl, $C_1$-$C_5$-Alkoxycarbonyl, $C_1$-$C_5$-Alkylcarbamoyl, Di-$C_1$-$C_5$-Alkylcarbamoyl oder $C_1$-$C_5$-Alkoxycarbamoyl steht.

Insbesondere stehen davon solche im Vordergrund, worin L für Methoxycarbonyl, Aethoxycarbonyl, Methylcarbamoyl, oder Methoxycarbamoyl steht.

Von den Verbindungen der Untergruppe e) sind diejenigen bevorzugt, in denen A für die Gruppe

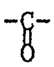 , $-CHOH$ , 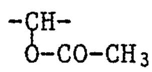 , 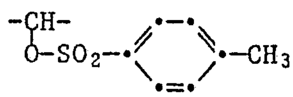 , 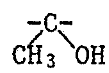 ,

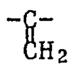 oder 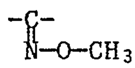 steht.

Eine besonders bevorzugte Gruppe von Wirkstoffen der Formel I wird von den Verbindugen gebildet, in denen $X^1$ Wasserstoff, Chlor, Methyl oder Methoxy, $X^2$ Wasserstoff, Methyl oder Methoxy, $X^3$ Wasserstoff, L die Gruppe $-COOR^{20}$ oder $-CONR^{20}R^{23}$, $R^1$, $R^2$, $R^3$ und $R^4$ unabhängig voneinander Wasserstoff oder Methyl und A die Gruppe

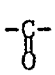 , $-CHOH-$ , 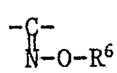 , 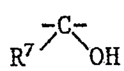 , 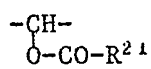 , 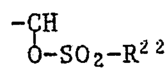

oder

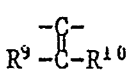

bedeuten, worin $R^6$, $R^7$, $R^9$ und $R^{10}$ unabhängig voneinander jeweils für Wasserstoff, $C_1$-$C_5$-Alkyl oder Phenyl, $R^{21}$ für $C_1$-$C_5$-Alkyl, $C_1$-$C_5$-Chloralkyl, $C_2$-$C_4$-Alkoxyalkyl oder Phenyl und $R^{22}$ für $C_1$-$C_5$-Alkyl oder gegebenenfalls durch Methyl, Chlor oder Nitro substituiertes Phenyl stehen.

Unter diesen Verbindungen stehen solche im Vordergrund des Interesses, in denen L für Carboxyl, Carbamoyl, $C_1$-$C_5$-Alkoxycarbonyl, $C_1$-$C_5$-Alkylcarbamoyl, Di-$C_1$-$C_5$-Alkylcarbamoyl oder $C_1$-$C_5$-Alkoxycarbamoyl und A für die Gruppe

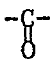 , -CHOH- , 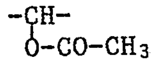 , 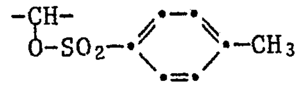 , 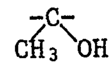 ,

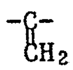 oder 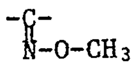

stehen, insbesondere aber hiervon diejenigen in welchen L für Methoxycarbonyl, Aethoxycarbonyl, Methylcarbamoyl, oder Methoxycarbamoyl steht.

Als bevorzugte Einzelverbindungen der vorliegenden Erfindung sind zu nennen:

1-(2,2-Dimethyl-3-oxo-indan-1-yl)-5-imidazolcarbonsäure-methylester,

1-(2,2-Dimethyl-4-oxo-tetralin-1-yl)-5-imidazolcarbonsäure-methylester,

1,3-trans-1-(3-Brom-2,2-dimethyl-4-oxo-tetralin-1-yl)-5-imidazolcarbonsäure-methylester,

1,3-cis-1-(3-Brom-2,2-dimethyl-4-oxo-tetralin-1-yl)-5-imidazolcarbonsäure-methylester,

1,4-trans-1-(2,2-Dimethyl-4-hydroxy-tetralin-1-yl)-5-imidazolcarbonsäure-methylester,

1,3-cis-1-(2,2-Dimethyl-3-hydroxy-indan-1-yl)-5-imidazolcarbonsäure-methylester,

1,3-trans-1-(2,2-Dimethyl-3-hydroxy-indan-1-yl)-5-imidazolcarbonsäure-methylester,

1,4-cis-1-(4-Acetoxy-2,2-dimethyl-tetralin-1-yl)-5-imidazolcarbonsäure-methylester,

1,4-trans-1-(4-Acetoxy-2,2-dimethyl-tetralin-1-yl)-5-imidazolcarbonsäure-methylester, und

1-(2,2-Dimethyl-3-methylidenyl-indan-1-yl)-5-imidazolcarbonsäure-methylester.

Die erfindungsgemässen Verbindungen der Formel I werden im allgemeinen nach den folgenden an sich bekannten Methoden hergestellt.

Man erhält die Verbindungen der Unterformel Ia

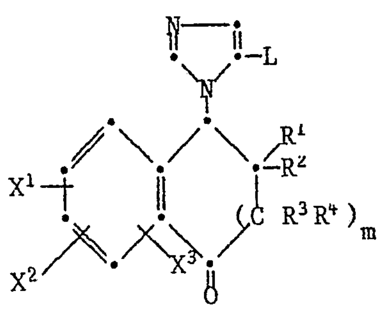

(Ia)

worin L für Cyan oder $C_1$-$C_5$-Alkoxycarbonyl steht und $R^1$, $R^2$, $R^3$, $R^4$, $X^1$, $X^2$, $X^3$ und m die unter Formel I gegebenen Bedeutungen haben, dadurch, dass man eine Verbindung der Formel II

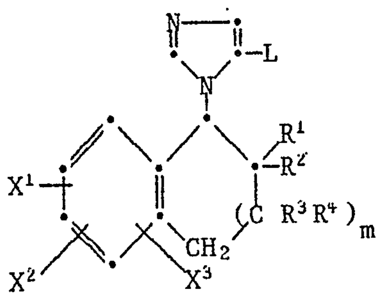

(II)

worin $R^1$, $R^2$, $R^3$, $R^4$, $X^1$, $X^2$, $X^3$, L und m die unter Formel Ia gegebenen Bedeutungen haben, mit einem Oxidationsmittel oxidiert.

Als besonders geeignete Oxidationsmittel haben sich für diese Umsetzung Kaliumpermanganat oder Ammoniumperoxodisulfat erwiesen. Vorzugsweise wird die Oxidation in verdünnten wässrigen Mineralsäuren wie Schwefelsäure, Salpetersäure oder Salzsäure ausgeführt. Bei Verwendung von Ammoniumperoxodisulfat kann das Oxidationsverfahren durch Zusatz von katalytischen Mengen Silbernitrat erleichtert und beschleunigt werden. Die Reaktionstemperaturen liegen zwischen 0°C und +120°C, vorzugsweise zwischen +40°C und +100°C, insbesondere zwischen +50°C und +80°C. Die Oxidation kann aber auch in organischen Lösungsmitteln unter Zuhilfenahme organischer Persäuren erfolgen. Geeignete Oxidationssysteme sind beispielsweise 3-Chlorperbenzoesäure, Perbenzoesäure oder Monoperphthalsäure in Methylenchlorid oder Chloroform. Gleiche Eignung haben Peressigsäure in Eisessig oder Perameisensäure in Ameisensäure. Die Reaktionstemperaturen liegen vorzugsweise zwischen -20°C und +60°C, insbesondere zwischen -10°C und +30°C. Die Ausgangsmaterialien der Formel II sind aus der EP-A-207 563 bekannt.

Die Verbindungen der Unterformel Ib

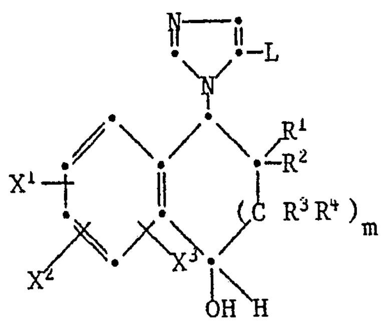

(Ib)

worin $R^1$, $R^2$, $R^3$, $R^4$, $X^1$, $X^2$, $X^3$, L und m die unter Formel Ia gegebenen Bedeutungen haben, werden aus den entsprechenden Verbindungen der Formel Ia vorzugsweise durch Reduktion der Carbonylfunktion erhalten.

Geeignete Reduktionsmittel sind durch ihre Selektivität bezüglich der Carbonylfunktion gekennzeichnet. Reduktionsmittel mit speziellem selektiven Reduktionsvermögen sind aus der Literatur bekannt. Im skizzierten Verfahren haben sich Natriumborhydrid oder Lithiumborhydrid als besonders geeignet erwiesen. Mit Vorteil führt man das Reduktionsverfahren in einem polaren inerten Lösungsmittel durch, wie Alkoholen, Aethern oder Kohlenwasserstoffen, beispielsweise Methanol, Aethanol, Isopropanol, Diäthyläther, Tetrahydrofuran, Dioxan, Cyclohexan, Benzol, Toluol oder Xylol. Die Reaktionstemperaturen liegen im allgemeinen zwischen -10°C und +70°C, vorzugsweise zwischen 0°C und +50°C.

Die Verbindungen der Unterformel Ic

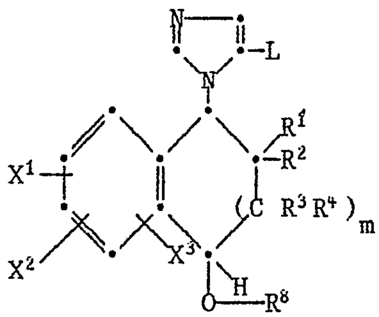

(Ic)

worin $R^1$, $R^2$, $R^3$, $R^4$, $X^1$, $X^2$, $X^3$, L und m die unter Formel Ia gegebene Bedeutung haben und $R^8$ für den Rest $-CO-R^{21}$ oder $-SO_2-R^{22}$ steht, werden durch Acylierung der Verbindungen der Formel Ib mit einem Acylhalogenid oder Säureanhydrid der Formeln III, IV, V oder VI

$Hal-CO-R^{21}$ (III)
$R^{21}-CO-O-CO-R^{21}$ (IV)
$Hal-SO_2-R^{22}$ (V)
$R^{22}-SO_2-O-SO_2-R^{22}$ (VI)

worin $R^{21}$ und $R^{22}$ die unter Formel I gegebenen Bedeutungen haben und Hal für Fluor, Chlor oder Brom, vorzugsweise aber Chlor steht, in Gegenwart einer Base erhalten.

Als Basen eignen sich sowohl anorganische als auch organische Basen. Bevorzugt sind solche Basen, die sich im mit Vorteil verwendeten inerten organischen Lösungsmittel lösen. Daher eignen sich besonders organische Amine zur Anwendung im genannten Verfahren. Solche Amine sind tertiäre aliphatische und aromatische Amine wie Trimethylamin, Triäthylamin, Tripropylamin, Chinuclidin, Pyridin, Chinolin und Isochinolin. Bevorzugte Amine sind Triäthylamin und insbesondere 1,4-Diazabicyclo[2.2.2]octan. Als anorganische Basen kommen in Betracht, Carbonate oder Hydrogencarbonate von Alkalimetallen oder Erdalkalimetallen. Als geeignete inerte organische Lösungsmittel bieten sich an: Aether oder Kohlenwasserstoffe wie Diäthyläther, Dioxan, Tetrahydrofuran, Benzol, Cyclohexan, Hexan, Xylole oder Toluol. Die Reaktionstemperaturen sind unkritisch und können in einem weiten Bereich variiert werden. Im allgemeinen liegen sie zwischen -20°C und +120°C, vorzugsweise zwischen 0°C und +80°C.

Die Verbindungen der Unterformel Id

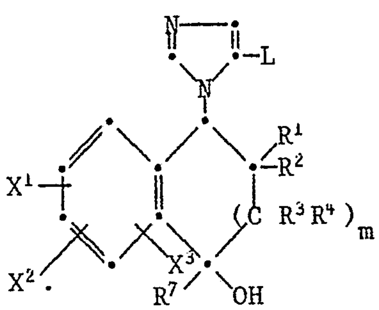

(Id)

worin $R^1$, $R^2$, $R^3$, $R^4$, $X^1$, $X^2$, $X^3$, L und m die unter Formel Ia gegebenen Bedeutungen haben und $R^7$ für $C_1$-$C_5$-Alkyl, $-CH_2-C_2$-$C_5$-Alkenyl, Benzyl oder Phenyl steht, werden aus den Verbindungen der Formel Ia durch Umsetzung mit einem Reagenz der Formel VII

$Hal-Mg-R^7$ (VII)

worin Hal für Brom oder Jod steht und $R^7$ die unter Formel Id gegebene Bedeutung hat, erhalten. Gewünschtenfalls kann die freie Hydroxylfunktion mit den Acylierungsreagenzien der Formeln III, IV, V oder VI in diejenigen Derivate der Formel I überführt werden, in denen A für die Gruppe

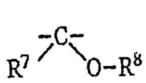

steht, worin $R^7$ und $R^8$ eine von Wasserstoff verschiedene Bedeutung haben. Die Reaktionsbedingungen der Acylierung entsprechen denen zur Herstellung der Verbindungen der Unterformel Ic.

Diese Reaktion (II+VII→Id) entspricht dem literaturbekannten Grignard-Typ und wird unter den dafür gebräuchlichen Reaktionsbedingungen in einem wasserfreien ätherischen Lösungsmittel durchgeführt. Typische Reaktionen verlaufen in Diäthyläther, Dioxan oder Tetrahydrofuran bei Temperaturen zwischen 0°C und +80°C, vorzugsweise zwischen +10°C und +50°C.

Setzt man die Verbindungen der Formel Id, worin $R^7$ $C_1$-$C_5$-Alkyl, Benzyl oder $-CH_2-C_2-C_5$-Alkenyl bedeutet, mit dehydratisierenden Reagenzien um, wie z.B. Trifluormethansulfonsäureanhydrid in Pyridin, so erhält man je nach Substitution des vicinalen Kohlenstoffatoms eine im Ring befindliche oder eine exocyclische Doppelbindung. Ist das vicinale Ringglied-Kohlenstoffatom durch kein Wasserstoffatom substituiert, so erhält man die exocyclische Doppelbindung, also Verbindungen der Formel Ie

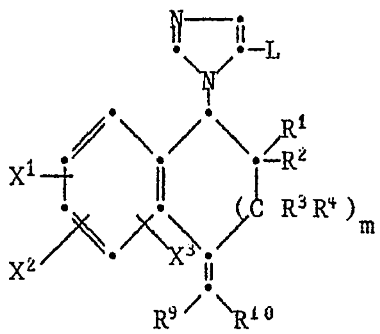

(Ie)

worin $R^1$, $R^2$, $R^3$, $R^4$, $X^1$, $X^2$, $X^3$, L und m die unter Formel Ia gegebene bedeutung haben und $R^9$ und $R^{10}$ die unter Formel I gegebenen Bedeutungen haben.

Ist das vicinale Ringglied-Kohlenstoffatom durch ein Wasserstoffatom substituiert und steht m für 1, so erhält man durch die beschriebene Dehydratisierung auch Zwischenprodukte des 1,2-Dihydronaphthalintyps der Formel VIII

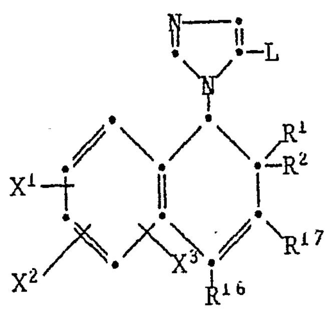

(VIII)

worin $R^1$, $R^2$, $R^3$, $R^4$, $X^1$, $X^2$, $X^3$, und L die unter Formel Ia gegebene Bedeutung haben und $R^{16}$ und $R^{17}$ für die unter Formel I gegebenen Definitionen stehen. Diese Zwischenprodukte der Formel VIII werden durch Oxidation mit Persäuren in die erfindungsgemässen Wirkstoffe der Unterformel If

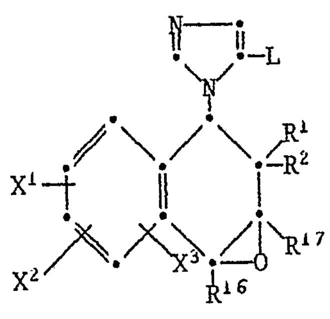

(If)

worin $R^1$, $R^2$, $R^3$, $R^4$, $X^1$, $X^2$, $X^3$, und L die unter Formel Ia gegebene Bedeutungen haben und $R^{16}$ und $R^{17}$ für die unter Formel I gegebenen Definitionen stehen, überführt.

Die Dehydratisierungsreaktionen (Id→Ic und Id→VIII) werden vorzugsweise bei Temperaturen zwischen -20°C und +50°C, insbesondere zwischen +10°C und +30°C durchgeführt. Bevorzugte Dehydratisierungssysteme sind zum Beispiel: Trifluormethansulfonsäureanhydrid in Pyridin bei -40°C bis -30°C; p-Toluolsulfonsäurechlorid in Pyridin bei +10°C bis +30°C; Methansulfonsäurechlorid in Triäthylamin und Aethylacetet bei -30°C bis -20°C; konzentrierte wässrige Bromwasserstoffsäure (48 %) bei +20°C bis +50°C.

Die Epoxidierung der Zwischenprodukte der Formel VIII wird durch Umsetzung mit organischen Persäuren erreicht. Beispiele für diese Reagenzien sind Perameisensäure, Peressigsäure, beziehungsweise Gemische von Ameisensäure oder Essigsäure mit Wasserstoffperoxid; Perbenzoesäure, 3-Chlorperbenzoesäure oder Monoperphthalsäure. Zur besseren Handhabbarkeit der Reaktion, wird mit Vorteil in einem organischen Lösungsmittel gearbeitet. Beispiele sind Carbonsäuren wie Ameisensäure oder Essigsäure oder chlorierte Kohlenwasserstoffe wie Tetrachlorkohlenstoff, Chloroform oder Methylenchlorid. Die Reaktionstemperaturen werden üblicherweise zwischen -20°C und +40°C, vorzugsweise zwischen -10°C und +20°C gehalten.

Die Verbindungen der Unterformel Ig

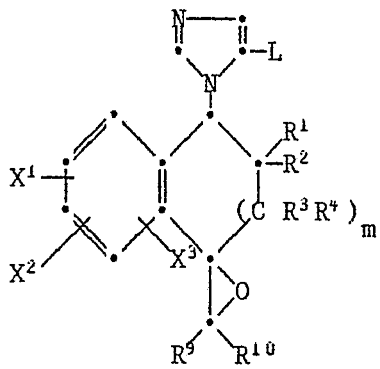

(Ig)

worin $R^1$, $R^2$, $R^3$, $R^4$, $R^9$, $R^{10}$, $X^1$, $X^2$, $X^3$, L und m die unter Formel Ie gegebenen Bedeutungen haben, werden durch Epoxidierung mit Persäuren aus den Verbindungen der Formel Ie erhalten.

Die Reaktionsbedingungen für die Reaktion Ie→Ig entsprechenden denen für die Umsetzung von VIII→If.

Die Verbindungen der Unterformel Ih

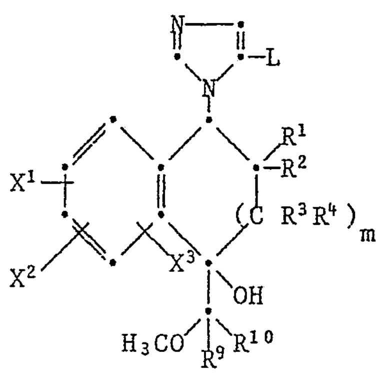

(Ih)

worin $R^1$, $R^2$, $R^3$, $R^4$, $R^9$, $R^{10}$, $X^1$, $X^2$, $X^3$, L und m die unter Formel Ie gegebene Bedeutung haben, erhält man durch Behandlung der Epoxide der Formel Ig mit Methanol in Gegenwart einer Base.

In einem typischen Reaktionsverlauf setzt man das Epoxid der Formel Ig in einer methanolischen Lösung von äquivalenten Mengen Alkalimetall, vorzugsweise Natrium oder Kalium, um. Die Reaktionstemperaturen liegen dabei im allgemeinen zwischen 0°C und +60°C, vorzugsweise zwischen +10°C und +30°C.

Die Verbindungen der Unterformel Ii

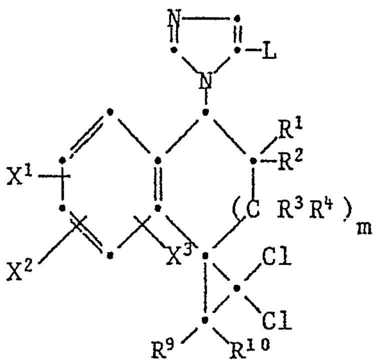

(Ii)

worin $R^1$, $R^2$, $R^3$, $R^4$, $R^9$, $R^{10}$, $X^1$, $X^2$, $X^3$, L und m die unter Formel Ie gegebenen Bedeutungen haben, erhält man die Verbindung der Formel Ie mit Dichlorcarben umsetzt.

Dichlorcarben wird in der Regel in Reaktionslösungen in situ erzeugt. In einer gebräuchlichen Verfahrensweise löst man die Verbindung der Formel Ie in Chloroform und fügt dieser Lösung als zweite Phase 30 %ige wässrige Alkalihydroxidlösung zu. Durch heftiges Rühren und Zusatz eines Phasentransfer-Katalysators wird die Addition des Dichlorcarbens an die exocyclische Doppelbindung begünstigt. Reaktionen dieses Typs sind in grosser Zahl aus der Literatur bekannt. Als Phasentransfer-Katalysatoren verwendet man im allgemeinen quaternäre Ammoniumsalze wie Tetraäthylammoniumbromid, Tetraäthylammoniumchlorid oder -jodid, Tetrabutylammoniumchlorid, -bromid oder -jodid, Triäthylbenzylammoniumchlorid, -bromid oder -jodid und weitere Tetraalkylammoniumhalogenide. Die Reaktionstemperaturen liegen üblicherweise zwischen -20°C und +80°C, vorzugsweise zwischen 0°C und +30°C.

Durch Behandlung mit 48 %iger Bromwasserstoffsäure in Dimethylsulfoxid bei Temperaturen zwischen +20°C und +130°C, vorzugsweise zwischen +50°C und +100°C erhält man aus den Verbindungen der Formel Ia solche Derivate, bei denen die α-Stellung bromiert ist, also für den Fall, dass m eins ist, steht dann $R^3$ oder $R^4$ für Brom.

Die Iminoverbindungen der Unterformel Ij

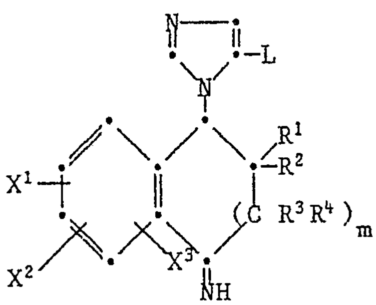

(Ij)

worin $R^1$, $R^2$, $R^3$, $R^4$, $X^1$, $X^2$, $X^3$, L und m die unter Formel Ia gegebenen Bedeutung haben, erhält man aus den Substanzen der Formel Ia durch Umsetzen mit der Verbindung der Formel IX

$(C_6H_5)_3P=N\text{-}Si(CH_3)_3$ (IX)

in einem inerten Kohlenwasserstoff wie Benzol, Toluol oder Xylol bei Temperaturen zwischen -20°C und +60°C, vorzugsweise zwischen 0°C und +20°C.

Die Verbindungen der Unterformel Ik

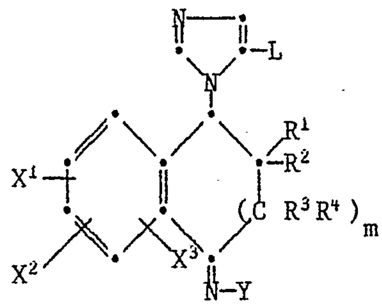

(Ik)

worin $R^1$, $R^2$, $R^3$, $R^4$, $X^1$, $X^2$, $X^3$, L und m die unter Formel Ia gegebenen Bedeutungen haben und Y für $C_1$-$C_5$-Alkyl, -$CH_2$-$C_2$-$C_5$-Alkenyl, Benzyl, Phenyl, -O-$R^6$ oder -$NR^{14}R^{15}$ steht, erhält man aus den Verbindungen der Formel Ia durch Kondensation mit den entsprechenden Aminverbindungen der Formel X

$H_2N$-Y (X)

worin Y für $C_1$-$C_5$-Alkyl, -$CH_2$-$C_2$-$C_5$-Alkenyl, Benzyl, Phenyl, -O-$R^6$ oder -$NR^{14}R^{15}$ steht und $R^6$, $R^{14}$ und $R^{15}$ die unter Formel I gegebenen Bedeutungen haben.

Die Reaktion (Ia→Ik) ist eine Kondensationsreaktion, welche unter Eliminierung von Wasser stattfindet. Die Reaktionsbedingungen für diese Kondensation sind in der Literatur in grosser Zahl beschrieben und unterscheiden sich zum Teil stark in der Wahl der Lösungsmittel und Reaktionstemperaturen.

Als besonders vorteilhaft hat es sich bei der vorliegenden Reaktion (Ia→Ik) erwiesen, ein polares Lösungsmittel wie Pyridin, bei Temperaturen zwischen -30° C und +40° C, vorzugsweise zwischen -10° C und +30° C, zu wählen.

Die zur Herstellung der verschiedenen Untergruppen Ia bis Ik benötigen Reagenzien und Ausgangsmaterialen sind in der Literatur beschrieben und grösstenteils im Handel erhältlich.

Die Verbindungen der Formeln Ia bis Ik können gewünschtenfalls durch übliche Derivatisierungsmethoden in die unter L in der Formel I umfassten Derivate überführt werden.

Bei der Derivatisierung wird der Alkoxycarbonylrest L in bekannter Weise modifiziert, beispielsweise durch Verseifung, Veresterung, Umesterung, Amidierung, Umamidierung oder Oximierung.

Die Cyanoverbindung (L = -CN) erhält man, indem man das Carbonsäureamid (L = -$CONH_2$) dehydratisiert. Als Mittel hierzu kommen beispielsweise Phosphorpentachlorid, Phosphoroxychlorid, Thienylchlorid, Phosphorpentoxid und Acetanhydrid in Frage. Die Reaktionstemperatur hängt von der Wahl des Dehydratisierungsmittels ab und liegt im allgemeinen zwischen +20° C und +120° C. Gegebenenfalls können auch inerte organische Lösungsmittel zugesetzt werden. Die Ester, Thiolester oder Amidderivate (X = -CO-D-$R^{20}$) lassen sich aus den Carbonsäuren (L = -COOH) oder den daraus erhältlichen aktivierten Verbindungen wie Säurehalogeniden oder Anhydriden oder gemischten Anhydriden durch Umsetzung mit den entsprechenden Alkoholen, Thiolen oder Aminverbindungen herstellen. Ester und Thiolester lassen sich ausserdem auch durch Alkylierung der Carbonsäuren mit Halogenalkylverbindungen in Gegenwart von Basen erhalten. Die Tetrazole sind durch Umsetzung der Nitrile (L = -CN) mit Aziden in organischen Lösungsmitteln wie Dimethylformamid oder Dimethylsulfoxid bei Temperaturen zwischen +20° C und +150° C erhältlich. Die anderen heterocyclischen Verbindungen der Formel I mit

L = 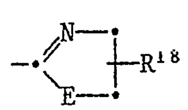 , 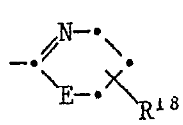 , 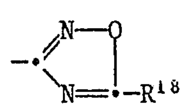 , 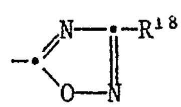 und

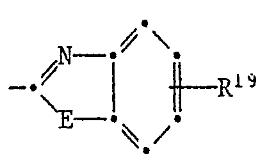

sind durch Umsetzung der entsprechenden Carbonsäuren, bzw. deren aktivierten Vertreter, wie ihren Säurehalogeniden, mit bifunktionellen Verbindungen wie Diaminen, Aminoalkohlen, Aminothiolen oder Amidoximen nach an sich bekannten Verfahren zugänglich. Die Thionoester (L = -CS-D-$R^{20}$) lassen sich aus dem entsprechenden Sauerstoffverbindungen durch Umsetzung mit Phosphorpentasulfid, vorteilhafterweise in Gegenwart von Basen, in organischen Lösungsmitteln wie Dimethylformamid, Acetonitril, Toluol oder Xylol erhalten. Die Amidoxime

($L = -\overset{NH_2}{\overset{|}{C}}=N-O-R^{18}$) werden aus den Nitrilen durch Umsetzung mit Hydroxylamin erhalten und können durch Umsetzung mit Alkylierungsmittel in die anderen Derivate ($R^{18}$ = Alkyl) umgewandelt werden.

Wird die Synthese von stereochemisch reinen Isomeren beabsichtigt, ist es empfehlenswert, stereoselektive Reaktionsschritte und -bedingungen zu wählen. Andererseits können reine Isomeren häufig durch übliche Trennungsmethoden aus dem Isomerengemisch abgetrennt werden, oder durch Verwendung von reinen optisch aktiven Ausgangsmaterialien in reiner Form erhalten werden.

Die Verbindungen der Formel I sind stabil und erfordern keine besonderen Vorsichtsmassnahmen für ihre Handhabung.

Bei Anwendung der Verbindung der Formel I in dem angegebenen Bereich der Aufwandmengen zeichnen sich diese Wirkstoffe durch gute selektiv-herbizide Eigenschaften aus, die sie ausgezeichnet zum Einsatz in Kulturen von Nutzpflanzen insbesondere Zuckerrüben, Sojabohnen, Getreide und Mais und ganz besonders Reis befähigen. Teilweise werden dabei auch Unkräuter geschädigt, welchen bisher nur mit Totalherbiziden beizukommen war. Werden Aufwandmengen angewendet, die den empfohlenen Bereich weit übersteigen, so werden alle behandelten Pflanzen so stark in ihrer Entwicklung geschädigt, dass sie absterben.

Die Erfindung betrifft auch herbizide Mittel, welche die neuen Verbindungen der Formel I enthalten. Ebenso erstreckt sich die Erfindung auf Verfahren zur Bekämpfung von Unkräutern durch die Anwendung dieser neuen Wirkstoffe.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulver, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na-oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen.

Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polyppropylenglykol, Aethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niederige, gegebenenfalls halogenierte Alkyl-, Benzyl- oder niedrige Hydroxyalkyl reste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1981;

H. Stache, "Tensid-Taschenbuch", 2. Aufl., C. Hanser Verlag, München, Wien, 1981;

M. and J. Ash. "Encyclopedia of Surfactants", Vol. I-III, Chemical Publishing Co., New York, 1980-81.

Die agrochemischen Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent).

Emulgierbare Konzentrate:
Aktiver Wirkstoff: 1 bis 20 %, bevorzugt 5 bis 10 %
oberflächenaktive Mittel: 5 bis 30 %, vorzugsweise 10 bis 20 %
flüssiges Trägermittel: 50 bis 94 %, vorzugsweise 70 bis 85 %

Stäube:
Aktiver Wirkstoff: 0,1 bis 10 %, vorzugsweise 0,1 bis 1 %
festes Trägermittel: 99,9 bis 90 %, vorzugsweise 99,9 bis 99 %

Suspensions-Konzentrate:
Aktiver Wirkstoff: 5 bis 75 %, vorzugsweise 10 bis 50 %
Wasser: 94 bis 25 %, vorzugsweise 88 bis 30 %
oberflächenaktives Mittel: 1 bis 40 %, vorzugsweise 2 bis 30 %

Benetzbares Pulver:
Aktiver Wirkstoff: 0,5 bis 90 %, vorzugsweise 1 bis 80 %
oberflächenaktives Mittel: 0,5 bis 20 %, vorzugsweise 1 bis 15 %
festes Trägermittel: 5 bis 99 %, vorzugsweise 15 bis 90 %

Granulate:
Aktiver Wirkstoff: 0,5 bis 30 %, vorzugsweise 3 bis 15 %
festes Trägermittel: 99,5 bis 70 %, vorzugsweise 97 bis 85 %.

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden.

Die nachfolgenden Beispiele illustrieren die vorliegende Erfindung, ohne eine Begrenzung hiervon darzustellen. Die Herstellungsbeispiele zeigen, wie man die neuen Verbindungen der Formel I erhalten kann. Die biologischen Beispiele und die Formulierungsbeispiele demonstrieren die Anwendung der Wirkstoffe für agrochemische Zwecke.

Herstellungsbeispiele:

Beispiel H1: 1-(2,2-Dimethyl-3-oxo-indan-1-yl)-5-imidazolcarbonsäure-methylester.

Eine Mischung von 30,0 g 1-(2,2-Dimethyl-indan-1-yl)-5-imidazolcarbonsäure-methylester, 150 ml Wasser und 17 g Schwefelsäure wird auf +80° C erwärmt. Innerhalb von 1,5 Stunden lässt man eine Lösung von 114 g Ammoniumperoxodisulfat zutropfen. Nach Beendigung der Reaktion wird die Lösung auf +7° C abgekühlt und durch Zusatz von 30 %iger Natronlauge auf pH 3 eingestellt. Man extrahiert die Lösung zweimal mit je 200 ml Methylenchlorid, trocknet die organische Phase über Natriumsulfat und dampft sie bis zur Trockne ein. Der Rückstand wird in einer kleinen Menge Methylenchlorid aufgenommen und mit Aether versetzt. Nachdem die entstehende Suspension filtriert worden ist, wird das Filtrat eingedampft und der Rückstand aus einem Aether/Hexan-Gemisch kristallisiert. Man erhält 19 g 1-(2,2-Dimethyl-3-oxo-indan-1-yl)-5-imidazolcarbonsäure-methylester mit einem Schmelzpunkt von 104-108° C.

Beispiel H2: 1-(2,2,3-Trimethyl-3-hydroxy-indan-1-yl)-5-imidazolcarbonsäure-methylester.

Aus 1,7 g Magnesiumspänen und 11 g Methyljodid in 40 ml Aether wird eine Lösung von Methylmagnesiumjodid zubereitet. Diese Lösung wird tropfenweise einer Lösung von 10 g 1-(2,2-Dimethyl-3-oxo-indan-1-yl)-5-carbonsäure-methylester in 300 ml Aether und 150 ml Tetrahydrofuran zugesetzt. Die breiige Suspension wird für weitere 10 Minuten gerührt und mit gesättigter Ammoniumsulfatlösung versetzt, bis eine Phasentrennung auftritt. Die organische Phase wird abgetrennt, über Natriumsulfat getrocknet und eingedampft. Der Rückstand wird chromatographisch an Kieselgel gereinigt. Man erhält so 4,2 g 1-(2,2,3-Trimethyl-3-hydroxy-indan-1-yl)-5-imidazolcarbonsäure-methylester mit einem Schmelzpunkt von 149-152° C.

Beispiel H3: 1-(2,2-Dimethyl-3-methyliden-indan-1-yl)-5-imidazol-carbonsäure-methylester.

Zu einer Lösung von 2,5 g 1-(2,2,3-Trimethyl-3-hydroxy-indan-1-yl)-5-imidazolcarbonsäure-methylester in 20 ml Pyridin lässt man bei einer Temperatur von -15° C 1,7 ml Trifluormethansulfonsäureanhydrid zutropfen. Nach einer Reaktionszeit von 10 Minuten wird die Mischung im Vakuum eingedampft und der Rückstand an Kieselgel mit einem Aether/Methylenchlorid-Gemisch (1:1) chromatographiert. Man erhält so 1,2 g 1-(2,2-Dimethyl-3-methyliden-indan-1-yl)-5-imidazol-carbonsäure-methylester in Form braunstichiger farbloser Kristalle mit einem Schmelzpunkt von 125-126° C.

Beispiel H4: 1-(2,2-Dimethyl-4-oxo-tetralin-1-yl)-5-imidazolcarbonsäure-methylester

142 g 1-(2,2-Dimethyl-tetralin-1-yl)-5-imidazolcarbonsäure-methylester werden in 1 Liter destilliertem Wasser suspendiert. Die Suspension wird auf +87° C erwärmt und unter Rühren innerhalb von 1,5 Stunden portionsweise mit insgesamt 335 g Ammoniumperoxodisulfat so versetzt, dass die Temperatur +90° C nicht überschritt. Nach Abkühlung der Reaktionsmischung auf +15° C wird der pH-Wert durch Zusatz von ungefähr 335 g 30%iger Natronlauge auf pH 4 eingestellt und mit 500 ml Methylenchlorid extra hiert. Die organische Phase wird abgetrennt, über Natriumsulfat getrocknet, mit Aktivkohle behandelt und eingedampft. Durch Kristallisation des Rückstandes aus Aether erhält man 42 g 1-(2,2-Dimethyl-4-oxo-tetralin-1-yl)-5-imidazolcarbonsäure-methylester mit einem Schmelzpunkt von 93-95° C.

Beispiel H5: 1-(3-Brom-2,2-dimethyl-4-oxo-tetralin-1-yl)-5-imidazolcarbonsäure-methylester

Zu einer Lösung von 3,0 g 1-(2,2-Dimethyl-4-oxo-tetralin-1-yl)-5-imidazolcarbonsäure-methylester in 20 ml Dimethylsulfoxid lässt man 3,0 g 48 % Bromwasserstoffsäure zutropfen. Die Reaktionsmischung wird für 30 Minuten auf +95° C erhitzt und nach dem Abkühlen auf 300 ml Eiswasser gegossen. Das Produkt wird dreimal mit je 100 ml Aether extrahiert. Die vereinigten organischen Phasen werden zweimal mit 2 %iger Natriumhydrogencarbonatlösung gewaschen, über Magnesiumsulfat getrocknet und eingedampft. Der Rückstand wird mit warmen Aether aufgenommen und auf +10° C abgekühlt. Der ausgefallene Niederschlag wird abgetrennt. Man erhält so 1,9 g des 1,3-trans-1-(3-Brom-2,2-dimethyl-4-oxo-tetralin-1-yl)-5-imidazolcarbonsäure-methylester mit einem Schmelzpunkt von 170-173° C. Aus der weiter eingedampften Mutterlauge erhält man durch fraktionierte Kristallisation 0,3 g 1,3-cis-1-(3-Brom-2,2-dimethyl-4-oxo-tetralin-1-yl)-5-imidazolcarbonsäure-methylester mit einem Schmelzpunkt von 176-178° C.

Beispiel H6: 1-(2,2-Dimethyl-4-hydroxy-tetralin-1-yl)-5-imidazolcarbonsäure-methylester.

Eine Lösung von 20,9 g 1-(2,2-Dimethyl-4-oxo-tetralin-1-yl)-5-imidazolcarbonsäure-methylester in 200 ml Methanol wird bei Raumtemperatur mit 4,0 g Natriumborhydrid versetzt und für 30 Minuten bei der gleichen Temperatur gerührt. Nach dem Eindampfen der Reaktionsmischung wird der Rückstand mit einem Aether/Methylenchlorid-Gemisch (1:1) an Kieselgel chromatographisch getrennt. Man erhält so 11,0 g 1,4-trans-1-(2,2-Dimethyl-4-hydroxy-tetralin-1-yl)-5-imidazolcarbonsäure-methylester mit einem Schmelzpunkt von 135-136° C und 3,0 g 1,4-cis-1-(2,2-Dimethyl-4-hydroxy-tetralin-1-yl)-5-imidazolcarbonsäure-methylester mit einem Schmelzpunkt von 179-180° C.

Beispiel H7: 1,4-trans-1-(4-Acetoxy-2,2-dimethyl-tetralin-1-yl)-5-imidazolcarbonsäure-methylester.

Eine Lösung von 1,8 g 1,4-trans-1-(2,2-Dimethyl-4-hydroxy-tetralin-1-yl)-5-imidazolcarbonsäure-methylester in 20 ml Tetrahydrofuran wird mit 1,0 g Triäthylamin und 0,8 g Acetylchlorid versetzt und für 30 Minuten bei Raumtemperatur gerührt. Das ausgefallene Triäthylammoniumchlorid wird abgetrennt, das Filtrat eingedampft und der Rückstand mit einem Aether/Hexan-Gemisch (2:1) an Kieselgel chromatographisch gereinigt. Man erhält so 1,0 g 1,4-trans-1-(4-Acetoxy-2,2-dimethyl-tetralin-1-yl)-5-imidazolcarbonsäure-methylester in Form eines farblosen Oeles.

Beispiel H8: 1,4-trans-1-(2,2-Dimethyl-4-methylsulfonyloxy-tetralin-1-yl)-5-imidazolcarbonsäure-methylester.

In 30 ml Toluol werden 1 g Triäthylamin und 1,8 g 1,4-trans-1-(2,2-Dimethyl-4-hydroxy-tetralin-1-yl)-5-imidazolcarbonsäure-methylester gelöst und mit 0.8 g Methansulfonsäurechlorid versetzt. Nach 30 Minuten Rühren bei +80°C wird auf Raumtemperatur abgekühlt, vom ausgefallenen Triäthylammoniumchlorid abfiltriert und das Filtrat eingedampft. Die anschliessende chromatographische Reinigung an Kieselgel mit Aether/Hexan (3:1) als Elutionsmittel ergibt 1,2 g 1,4-trans-1-(2,2-Dimethyl-4-methylsulfonyloxy-tetralin-1-yl)-5-imidazolcarbonsäure-methylester als farbloses Harz.

Beispiel H9: 1-(3,4-Epoxy-2,2-dimethyl-tetralin-1-yl)-5-imidazolcarbonsäure-methylester.

a) 1-(1,2-Dihydro-2,2-dimethyl-naphthalin-1-yl)-5-imidazolcarbonsäure-methylester.

0,9 g 1,4-trans-1-(2,2-Dimethyl-4-methylsulfonyloxy-tetralin-1-yl)-5- imidazolcarbonsäure-methylester werden in 5 ml Pyridin für 30 Minuten bei +60°C gerührt. Anschliessend wird das Pyridin am Vakuum weitgehend abgedampft und der Rückstand an Kieselgel chromatographisch gereinigt, wobei als Elutionsmittel ein Metyhlenchlorid/Aether-Gemisch (30:1) verwendet wird. Man erhält 0,4 g 1-(1,2-Dihydro-2,2-dimethyl-naphthalin-1-yl)-5-imidazolcarbonsäure-methylester vom Schmelzpunkt 60-61°C.

b) Eine Lösung von 8,5 g 1-(1,2-Dihydro-2,2-dimethyl-naphthalin-1-yl)-5-imidazolcarbonsäure-methylester in 150 ml Methylenchlorid wird mit 9,4 g 55 %-iger 3-Chlorperbenzoesäure versetzt und für 2 Stunden bei Raumtemperatur gerührt. Der Niederschlag wird abgetrennt und das Filtrat zweimal mit je 50 ml Natriumhydrogencarbonat-Lösung (5 %) gewaschen, über Magnesiumsulfat getrocknet und eingedampft. Durch Chromatographie des Rückstandes an Kieselgel [Elutionsmittel: Aether/Hexan (3:1)] liefert 1,7 g eines Gemisches von 1,4-cis-1-(3,4-Epoxy-2,2-dimethyl-tetralin-1-yl)-5-imidazol-carbonsäure-methylester und 1,4-trans-1-(3,4-Epoxy-2,2-dimethyl-tetralin-1-yl)-5-imidazolcarbonsäure-methylester im Verhältnis 1:1 (Smp.: 60-68°C)

Beispiel H10: 1-(1,2-Dihydro-2,2-dimethyl-naphthalin-1-yl)-5-imidazolcarbonsäure-methylester.

Einstufiges Verfahren zur Herstellung des nach Beispielen H8 und H9 a) hergestellten Zwischenprodukts.

Eine Mischung von 1,8 g Methansulfonsäurechlorid und 5,2 g 1,4-trans-1-(2,2-Dimethyl-4-hydroxy-tetralin-1-yl)-5-imidazolcarbonsäure-methylester in 100 m Aethylacetat wird bei Raumtemperatur tropfenweise mit 5 ml Triäthylamin versetzt. Nach einer Reaktionszeit von einer Stunde wird der entstandene Niederschlag abgetrennt und die überstehende Lösung eingedampft. Der Rückstand wird an Kieselgel mit einem Aether/Methylenchlorid-Gemisch (1:1) chromatographisch gereinigt. Man erhält 2,5 g 1-(1,2-Dihydro-2,2-dimethyl-naphthalin-1-yl)-5-imidazolcarbonsäure-methylester mit einem Schmelzpunkt von 60-62°C.

Beispiel H11: 1-(2,2-Dimethyl-4-oxo-tetralin-1-yl)-5-imidazolcarbonsäure-methylester

Zu einer auf 0°C gekühlten Lösung von 7,1 g 1-(2,2-Dimethyl-tetralin-1-yl)-5-imidazolcarbonsäure-methylester in 200 ml Methylenchlorid setzt man 10,6 g 3-Chlorperbenzoesäure zu. Die Mischung wird bei Raumtemperatur für weitere 72 Stunden gerührt. Anschliessend setzt man eine Lösung von 7,5 g Natriumhydrogencarbonat in 100 ml Wasser zu. Die organische Phase wird abgetrennt, mit Wasser gewaschen, getrocknet und eingedampft. Der Rückstand wird säulenchromatographisch an Kieselgel mit Chloroform als Elutionsmittel gereinigt. Die produkthaltige Fraktion wird erneut an Kieselgel mit einem Chloroform/Methanol-Gemisch (99,5:0,5) chromatographiert. Man erhält 0,3 g 1-(2,2-Dimethyl-4-oxo-tetralin-1-yl)-5-imidazolcarbonsäure-methylester als Feststoff.

Die in den anschliessenden Tabellen 1 bis 15 aufgelisteten Zwischen-und Endprodukte werden in analoger Weise erhalten.

Tabelle 1:

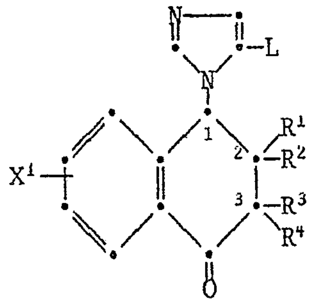

| Verb. Nr. | $R^1$ | $R^2$ | $R^3$ | $R^4$ | $X^1$ | L | physikalische Daten |
|---|---|---|---|---|---|---|---|
| 1.01 | H | H | H | H | H | $COOCH_3$ | Smp. 90-92°C |
| 1.02 | $C_2H_5$ | $C_2H_5$ | $CH_3$ | H | H | $COOCH_3$ | |
| 1.03 | $CH_3$ | H | H | H | H | $COOCH_3$ | 1,2-cis |
| 1.04 | $CH_3$ | H | H | H | H | $COOCH_3$ | 1,2-trans |
| 1.05 | $CH_3$ | $CH_3$ | H | H | H | $COOCH_3$ | Smp. 103-105°C |
| 1.06 | $CH_3$ | $CH_3$ | H | H | H | $COOC_2H_5$ | |
| 1.07 | $CH_3$ | $CH_3$ | H | H | H | $COOC_4H_9$-t | Harz |
| 1.08 | $CH_3$ | $CH_3$ | $CH_3$ | H | H | $COOCH_3$ | 1,3-cis |
| 1.09 | $CH_3$ | $CH_3$ | $CH_3$ | H | H | $COOCH_3$ | 1,3-trans |
| 1.10 | H | H | $CH_3$ | H | H | $COOCH_3$ | 1,3-cis |
| 1.11 | H | H | $CH_3$ | H | H | $COOCH_3$ | 1,3-trans |
| 1.12 | H | H | $CH_3$ | $CH_3$ | H | $COOCH_3$ | |
| 1.13 | $CH_3$ | H | $CH_3$ | H | H | $COOCH_3$ | 2,3-cis |
| 1.14 | $CH_3$ | H | $CH_3$ | H | H | $COOCH_3$ | 2,3-trans |
| 1.15 | H | $-(CH_2)_3-$ | | H | H | $COOCH_3$ | |
| 1.16 | $CH_3$ | $CH_3$ | H | H | H | $CONHCH_3$ | |
| 1.17 | $CH_3$ | $CH_3$ | H | H | H | $CONH-OCH_3$ | |
| 1.18 | $CH_3$ | $CH_3$ | H | H | 7-$CH_3$ | $COOCH_3$ | |
| 1.19 | $CH_3$ | $CH_3$ | H | H | 7-Cl | $COOCH_3$ | |
| 1.20 | $CH_3$ | $CH_3$ | H | H | 5-Cl | $COOCH_3$ | |
| 1.21 | $CH_3$ | $CH_3$ | Br | H | H | $COOCH_3$ | 1,3-trans, Smp. 170-173°C |
| 1.22 | $CH_3$ | $CH_3$ | Br | H | H | $COOCH_3$ | 1,3-cis, Smp. 176-178°C |
| 1.23 | $CH_3$ | $CH_3$ | H | H | H | COOH | Smp. 240°C (Zers.) |

Tabelle 2:

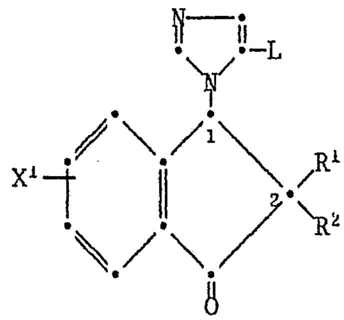

| Verb. Nr. | $R^1$ | $R^2$ | $X^1$ | L | physikalische Daten |
|---|---|---|---|---|---|
| 2.01 | $CH_3$ | $CH_3$ | H | $COOCH_3$ | Smp. 119-121°C |
| 2.02 | $CH_3$ | $CH_3$ | H | $COOC_2H_5$ | |
| 2.03 | $C_4H_9$-t | H | H | $COOCH_3$ | |
| 2.04 | $CH_3$ | $CH_3$ | H | $CONHCH_3$ | |
| 2.05 | $CH_3$ | $CH_3$ | H | $CONH-OCH_3$ | |
| 2.06 | $C_2H_5$ | $C_2H_5$ | H | $COOCH_3$ | |
| 2.07 | $CH_3$ | $CH_3$ | 6-$CH_3$ | $COOCH_3$ | |
| 2.08 | $CH_3$ | $CH_3$ | 6-Cl | $COOCH_3$ | |
| 2.09 | $CH_3$ | $CH_3$ | 6-$OCH_3$ | $COOCH_3$ | |
| 2.10 | $-(CH_2)_4-$ | | H | $COOCH_3$ | |
| 2.11 | $-(CH_2)_5-$ | | H | $COOCH_3$ | |

Tabelle 3:

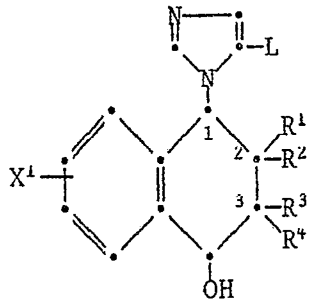

| Verb. Nr. | $R^1$ | $R^2$ | $R^3$ | $R^4$ | $X^1$ | L | physikalische Daten |
|---|---|---|---|---|---|---|---|
| 3.01 | H | H | H | H | H | $COOCH_3$ | 1,4-trans, Smp. 131-134°C |
| 3.02 | $C_2H_5$ | $C_2H_5$ | $CH_3$ | H | H | $COOCH_3$ | |
| 3.03 | $CH_3$ | H | H | H | H | $COOCH_3$ | 1,2-cis |
| 3.04 | $CH_3$ | H | H | H | H | $COOCH_3$ | 1,2-trans |
| 3.05 | $CH_3$ | $CH_3$ | H | H | H | $COOCH_3$ | 1,4-trans, Smp. 135-136°C |
| 3.06 | $CH_3$ | $CH_3$ | H | H | H | $COOC_2H_5$ | |
| 3.07 | $CH_3$ | $CH_3$ | H | H | H | $COOC_4H_9$-t | |
| 3.08 | $CH_3$ | $CH_3$ | $CH_3$ | H | H | $COOCH_3$ | 1,3-cis |
| 3.09 | $CH_3$ | $CH_3$ | $CH_3$ | H | H | $COOCH_3$ | 1,3-trans |
| 3.10 | H | H | $CH_3$ | H | H | $COOCH_3$ | 1,3-cis |
| 3.11 | H | H | $CH_3$ | H | H | $COOCH_3$ | 1,3-trans |
| 3.12 | H | H | $CH_3$ | $CH_3$ | H | $COOCH_3$ | |
| 3.13 | $CH_3$ | H | $CH_3$ | H | H | $COOCH_3$ | 2,3-cis |
| 3.14 | $CH_3$ | H | $CH_3$ | H | H | $COOCH_3$ | 2,3-trans |
| 3.15 | H | $-(CH_2)_3-$ | | H | H | $COOCH_3$ | |
| 3.16 | $CH_3$ | $CH_3$ | H | H | H | $CONHCH_3$ | |
| 3.17 | $CH_3$ | $CH_3$ | H | H | H | $CONH-OCH_3$ | |
| 3.18 | $CH_3$ | $CH_3$ | H | H | 7-$CH_3$ | $COOCH_3$ | |
| 3.19 | $CH_3$ | $CH_3$ | H | H | 7-Cl | $COOCH_3$ | |
| 3.20 | $CH_3$ | $CH_3$ | H | H | 5-Cl | $COOCH_3$ | |
| 3.21 | $CH_3$ | $CH_3$ | H | H | H | $COOCH_3$ | 1,4-cis, Smp. 179-180°C |
| 3.22 | H | H | H | H | H | $COOCH_3$ | 1,4-cis, Smp. 150-154°C |

Tabelle 4:

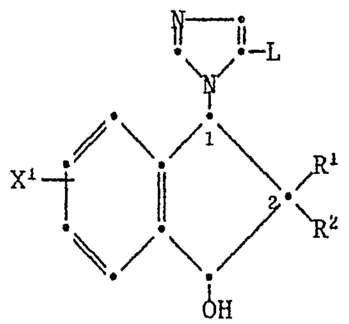

| Verb. Nr. | $R^1$ | $R^2$ | $X^1$ | L | physikalische Daten |
|---|---|---|---|---|---|
| 4.01 | $CH_3$ | $CH_3$ | H | $COOCH_3$ | 1,3-cis, Smp. 194-195°C |
| 4.02 | $CH_3$ | $CH_3$ | H | $COOC_2H_5$ | |
| 4.03 | $C_4H_9$-t | H | H | $COOCH_3$ | |
| 4.04 | $CH_3$ | $CH_3$ | H | $CONHCH_3$ | |
| 4.05 | $CH_3$ | $CH_3$ | H | $CONH-OCH_3$ | |
| 4.06 | $C_2H_5$ | $C_2H_5$ | H | $COOCH_3$ | |
| 4.07 | $CH_3$ | $CH_3$ | 6-$CH_3$ | $COOCH_3$ | |
| 4.08 | $CH_3$ | $CH_3$ | 6-Cl | $COOCH_3$ | |
| 4.09 | $CH_3$ | $CH_3$ | 6-$OCH_3$ | $COOCH_3$ | |
| 4.10 | $-(CH_2)_4-$ | | H | $COOCH_3$ | |
| 4.11 | $-(CH_2)_5-$ | | H | $COOCH_3$ | |
| 4.12 | $CH_3$ | $CH_3$ | H | $COOCH_3$ | 1,3-trans, Smp. 145-146°C |

Tabelle 5:

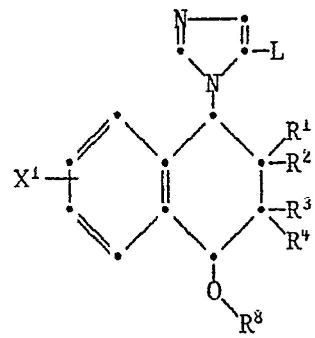

| Verb. Nr. | $R^1$ | $R^2$ | $R^3$ | $R^4$ | $X^1$ | $R^8$ | L | phys. Daten |
|---|---|---|---|---|---|---|---|---|
| 5.01 | H | H | H | H | H | $COCH_3$ | $COOCH_3$ | 1,4-cis |
| 5.02 | H | H | H | H | H | $COCH_3$ | $COOCH_3$ | 1,4-trans |
| 5.03 | H | H | H | H | H | $COCH_2Cl$ | $COOCH_3$ | 1,4-trans |
| 5.04 | H | H | H | H | H | $COCH_2-OCH_3$ | $COOCH_3$ | 1,4-trans |
| 5.05 | H | H | H | H | H | $COC_6H_5$ | $COOCH_3$ | |
| 5.06 | $CH_3$ | $CH_3$ | H | H | H | $COCH_3$ | $COOCH_3$ | 1,4-cis, Oel |
| 5.07 | $CH_3$ | $CH_3$ | H | H | H | $COCH_3$ | $COOCH_3$ | 1,4-trans, Oel |
| 5.08 | $CH_3$ | $CH_3$ | H | H | H | $COC_2H_5$ | $COOCH_3$ | |
| 5.09 | $CH_3$ | $CH_3$ | H | H | H | $COCH_2Cl$ | $COOCH_3$ | |
| 5.10 | $CH_3$ | $CH_3$ | H | H | H | $COCH_3$ | $COOC_2H_5$ | |
| 5.11 | $CH_3$ | $CH_3$ | H | H | H | $COCH_3$ | $COOC_4H_9$-t | |
| 5.12 | $CH_3$ | H | H | H | H | $COCH_3$ | $COOCH_3$ | |
| 5.13 | $CH_3$ | H | H | H | H | $COCH_2Cl$ | $COOCH_3$ | |
| 5.14 | $CH_3$ | H | H | H | H | $COCH_2-OCH_3$ | $COOCH_3$ | |
| 5.15 | $CH_3$ | H | H | H | H | $COC_6H_5$ | $COOCH_3$ | |
| 5.16 | H | H | H | H | H | $SO_2CH_3$ | $COOCH_3$ | 1,4-trans |
| 5.17 | H | H | H | H | H | $SO_2-C_6H_4-4-CH_3$ | $COOCH_3$ | 1,4-cis |
| 5.18 | H | H | H | H | H | $SO_2CH_3$ | $COOCH_3$ | 1,4-cis |
| 5.19 | H | H | H | H | H | $SO_2-C_6H_4-4-CH_3$ | $COOCH_3$ | 1,4-trans |
| 5.20 | H | H | H | H | H | $SO_2-C_6H_5$ | $COOCH_3$ | |
| 5.21 | $CH_3$ | $CH_3$ | H | H | H | $SO_2-C_6H_4-4-CH_3$ | $COOCH_3$ | 1,4-trans, Oel |
| 5.22 | $CH_3$ | $CH_3$ | H | H | H | $SO_2-C_6H_4-4-CH_3$ | $COOCH_3$ | 1,4-cis, Oel |

Tabelle 5 (Fortsetzung)

| Verb. Nr. | $R^1$ | $R^2$ | $R^3$ | $R^4$ | $X^1$ | $R^8$ | L | phys. Daten |
|---|---|---|---|---|---|---|---|---|
| 5.23 | $CH_3$ | $CH_3$ | H | H | H | $SO_2CH_3$ | $COOCH_3$ | |
| 5.24 | $CH_3$ | $CH_3$ | H | H | H | $SO_2-C_6H_5$ | $COOCH_3$ | |
| 5.25 | $CH_3$ | $CH_3$ | H | H | H | $SO_2CH_3$ | $COOC_2H_5$ | |
| 5.26 | $CH_3$ | $CH_3$ | H | H | H | $SO_2CH_3$ | $COOC_4H_9$-t | |
| 5.27 | $CH_3$ | H | H | H | H | $SO_2CH_3$ | $COOCH_3$ | |
| 5.28 | $CH_3$ | H | H | H | H | $SO_2-C_6H_4-4-CH_3$ | $COOCH_3$ | |
| 5.29 | $CH_3$ | H | H | H | H | $SO_2-C_6H_5$ | $COOCH_3$ | |
| 5.30 | $CH_3$ | H | H | H | H | $SO_2-C_6H_4-2-NO_2$ | $COOCH_3$ | |

Tabelle 6:

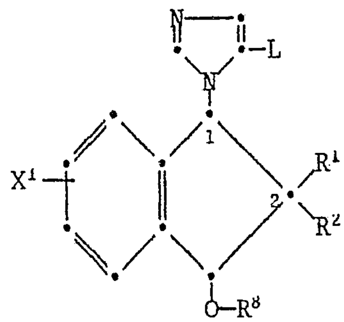

| Verb. Nr. | $R^1$ | $R^2$ | $X^1$ | $R^8$ | L | phys. Daten |
|---|---|---|---|---|---|---|
| 6.01 | $CH_3$ | $CH_3$ | H | $COCH_3$ | $COOCH_3$ | 1,3-trans |
| 6.02 | $CH_3$ | $CH_3$ | H | $COCH_3$ | $COOCH_3$ | 1,3-cis |
| 6.03 | $CH_3$ | $CH_3$ | H | $COC_2H_5$ | $COOCH_3$ | Harz |
| 6.04 | $CH_3$ | $CH_3$ | H | $COCH_2Cl$ | $COOCH_3$ | |
| 6.05 | $CH_3$ | $CH_3$ | H | $COCH_3$ | $COOC_2H_5$ | |
| 6.06 | $CH_3$ | H | H | $COCH_3$ | $COOCH_3$ | |
| 6.07 | $CH_3$ | H | H | $COCH_2Cl$ | $COOCH_3$ | |
| 6.08 | $CH_3$ | H | H | $COCH_2-OCH_3$ | $COOCH_3$ | |
| 6.09 | $CH_3$ | $CH_3$ | H | $SO_2CH_3$ | $COOCH_3$ | |
| 6.10 | $CH_3$ | $CH_3$ | H | $SO_2-C_6H_4-4-CH_3$ | $COOCH_3$ | |
| 6.11 | $CH_3$ | $CH_3$ | H | $SO_2-C_6H_5$ | $COOCH_3$ | |
| 6.12 | $CH_3$ | $CH_3$ | H | $SO_2-C_6H_4-2-NO_2$ | $COOCH_3$ | |
| 6.13 | $CH_3$ | $CH_3$ | H | $SO_2CH_3$ | $COOC_2H_5$ | |
| 6.14 | $CH_3$ | H | H | $SO_2CH_3$ | $COOCH_3$ | |
| 6.15 | $CH_3$ | H | H | $SO_2-C_6H_4-4-CH_3$ | $COOCH_3$ | |
| 6.16 | $CH_3$ | H | H | $SO_2-C_6H_5$ | $COOCH_3$ | |

Tabelle 7:

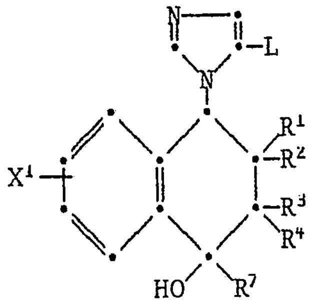

| Verb. Nr. | $R^1$ | $R^2$ | $R^3$ | $R^4$ | $X^1$ | $R^7$ | L | phys. Daten |
|---|---|---|---|---|---|---|---|---|
| 7.01 | H | H | H | H | H | $CH_3$ | $COOCH_3$ | |
| 7.02 | H | H | H | H | H | $C_2H_5$ | $COOCH_3$ | |
| 7.03 | H | H | H | H | H | $C_3H_7$-n | $COOCH_3$ | |
| 7.04 | H | H | H | H | H | $C_3H_7$-i | $COOCH_3$ | |
| 7.05 | H | H | H | H | H | $C_6H_5$ | $COOCH_3$ | |
| 7.06 | $CH_3$ | $CH_3$ | H | H | H | $C_2H_5$ | $COOCH_3$ | |
| 7.07 | $CH_3$ | $CH_3$ | H | H | H | $CH_3$ | $COOCH_3$ | Smp. 186-189°C |
| 7.08 | $CH_3$ | $CH_3$ | H | H | H | $C_3H_7$-i | $COOCH_3$ | |
| 7.09 | $CH_3$ | $CH_3$ | H | H | H | $C_3H_7$-n | $COOCH_3$ | |
| 7.10 | $CH_3$ | $CH_3$ | H | H | H | $CH_3$ | $COOC_2H_5$ | |
| 7.11 | $CH_3$ | $CH_3$ | H | H | H | $CH_3$ | $COOC_4H_9$-t | |
| 7.12 | $CH_3$ | H | H | H | H | $CH_3$ | $COOCH_3$ | |
| 7.13 | $CH_3$ | H | H | H | H | $C_6H_5$ | $COOCH_3$ | |
| 7.14 | $CH_3$ | H | H | H | H | $C_3H_7$-i | $COOCH_3$ | |
| 7.15 | $CH_3$ | H | H | H | H | $C_3H_7$-n | $COOCH_3$ | |

Tabelle 8:

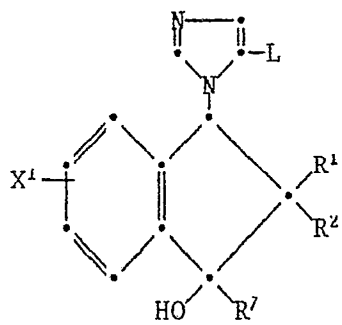

| Verb. Nr. | $R^1$ | $R^2$ | $X^1$ | $R^7$ | L | phys. Daten |
|---|---|---|---|---|---|---|
| 8.01 | $CH_3$ | $CH_3$ | H | $C_2H_5$ | $COOCH_3$ | |
| 8.02 | $CH_3$ | $CH_3$ | H | $CH_3$ | $COOCH_3$ | Smp. 149-152°C |
| 8.03 | $CH_3$ | $CH_3$ | H | $C_3H_7$-n | $COOCH_3$ | |
| 8.04 | $CH_3$ | $CH_3$ | H | $C_3H_7$-i | $COOCH_3$ | |
| 8.05 | $CH_3$ | $CH_3$ | H | $C_3H_7$-i | $COOC_2H_5$ | |
| 8.06 | $CH_3$ | H | H | $C_3H_7$-i | $COOCH_3$ | |
| 8.07 | $CH_3$ | H | H | $C_2H_5$ | $COOCH_3$ | |
| 8.08 | $CH_3$ | H | H | $CH_3$ | $COOCH_3$ | |
| 8.09 | $CH_3$ | H | H | $CH_3$ | $COOCH_3$ | 1,2-cis |
| 8.10 | $CH_3$ | H | H | $CH_3$ | $COOCH_3$ | 1,2-trans |

Tabelle 9:

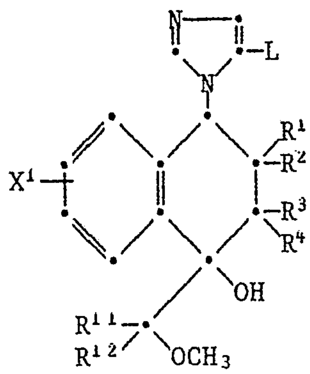

| Verb. Nr. | $R^1$ | $R^2$ | $R^3$ | $R^4$ | $X^1$ | $R^{11}$ | $R^{12}$ | L | phys. Daten |
|---|---|---|---|---|---|---|---|---|---|
| 9.01 | H | H | H | H | H | H | H | $COOCH_3$ | |
| 9.02 | H | H | H | H | H | $CH_3$ | $CH_3$ | $COOCH_3$ | |
| 9.03 | H | H | H | H | H | $C_2H_5$ | H | $COOCH_3$ | |
| 9.04 | H | H | H | H | H | $C_6H_5$ | H | $COOCH_3$ | |
| 9.05 | H | H | H | H | H | $C_2H_5$ | $C_2H_5$ | $COOCH_3$ | |
| 9.06 | $CH_3$ | $CH_3$ | H | H | H | H | H | $COOCH_3$ | |
| 9.07 | $CH_3$ | $CH_3$ | H | H | H | $CH_3$ | $CH_3$ | $COOCH_3$ | |
| 9.08 | $CH_3$ | $CH_3$ | H | H | H | $C_2H_5$ | $C_6H_5$ | $COOCH_3$ | |
| 9.09 | $CH_3$ | $CH_3$ | H | H | H | $C_6H_5$ | $C_6H_5$ | $COOCH_3$ | |
| 9.10 | $CH_3$ | $CH_3$ | H | H | H | H | H | $COOC_2H_5$ | |
| 9.11 | $CH_3$ | $CH_3$ | H | H | H | H | H | $COOC_4H_9$-t | |
| 9.12 | $CH_3$ | H | H | H | H | $CH_3$ | $CH_3$ | $COOCH_3$ | |
| 9.13 | $CH_3$ | H | H | H | H | H | H | $COOCH_3$ | |
| 9.14 | $CH_3$ | H | H | H | H | $C_6H_5$ | $C_6H_5$ | $COOCH_3$ | |
| 9.15 | $CH_3$ | H | H | H | H | $C_6H_5$ | H | $COOCH_3$ | |

Tabelle 10:

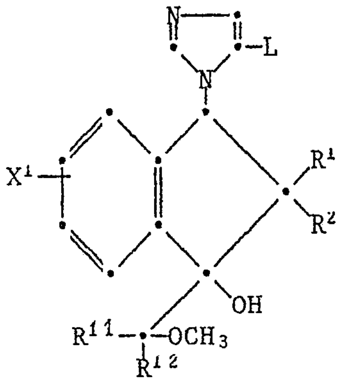

| Verb. Nr. | $R^1$ | $R^2$ | $X^1$ | $R^{11}$ | $R^{12}$ | L | phys. Daten |
|---|---|---|---|---|---|---|---|
| 10.01 | $CH_3$ | $CH_3$ | H | H | H | $COOCH_3$ | |
| 10.02 | $CH_3$ | $CH_3$ | H | $CH_3$ | $CH_3$ | $COOCH_3$ | |
| 10.03 | $CH_3$ | $CH_3$ | H | $C_6H_5$ | $C_6H_5$ | $COOCH_3$ | |
| 10.04 | $CH_3$ | $CH_3$ | H | $C_6H_5$ | H | $COOCH_3$ | |
| 10.05 | $CH_3$ | $CH_3$ | H | H | H | $COOC_2H_5$ | |
| 10.06 | $CH_3$ | H | H | $CH_3$ | $CH_3$ | $COOCH_3$ | |
| 10.07 | $CH_3$ | H | H | H | H | $COOCH_3$ | |
| 10.08 | $CH_3$ | H | H | $C_2H_5$ | H | $COOCH_3$ | |

Tabelle 11:

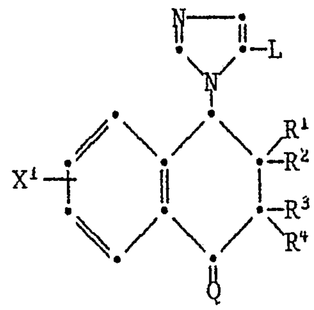

| Verb. Nr. | $R^1$ | $R^2$ | $R^3$ | $R^4$ | $X^1$ | Q | L | phys. Daten |
|---|---|---|---|---|---|---|---|---|
| 11.01 | H | H | H | H | H | $CH_2$ | $COOCH_3$ | |
| 11.02 | H | H | H | H | H | $C(CH_3)_2$ | $COOCH_3$ | |
| 11.03 | H | H | H | H | H | $CH-C_2H_5$ | $COOCH_3$ | |
| 11.04 | H | H | H | H | H | $CH-C_6H_5$ | $COOCH_3$ | |
| 11.05 | H | H | H | H | H | $C(C_2H_5)_2$ | $COOCH_3$ | |
| 11.06 | $CH_3$ | $CH_3$ | H | H | H | $C(CH_3)_2$ | $COOCH_3$ | |
| 11.07 | $CH_3$ | $CH_3$ | H | H | H | $CH_2$ | $COOCH_3$ | |
| 11.08 | $CH_3$ | $CH_3$ | H | H | H | $CH-C_2H_5$ | $COOCH_3$ | |
| 11.09 | $CH_3$ | $CH_3$ | H | H | H | $C(C_6H_5)_2$ | $COOCH_3$ | |
| 11.10 | $CH_3$ | $CH_3$ | H | H | H | $CH_2$ | $COOC_2H_5$ | |
| 11.11 | $CH_3$ | $CH_3$ | H | H | H | $CH_2$ | $COOC_4H_9$-t | |
| 11.12 | $CH_3$ | H | H | H | H | $C(CH_3)_2$ | $COOCH_3$ | |
| 11.13 | $CH_3$ | H | H | H | H | $CH_2$ | $COOCH_3$ | |
| 11.14 | $CH_3$ | H | H | H | H | $C(C_6H_5)_2$ | $COOCH_3$ | |
| 11.15 | $CH_3$ | H | H | H | H | $CH-C_6H_5$ | $COOCH_3$ | |
| 11.16 | H | H | H | H | H | NH | $COOCH_3$ | |
| 11.17 | H | H | H | H | H | $N-NH_2$ | $COOCH_3$ | |
| 11.18 | H | H | H | H | H | $N-CH_3$ | $COOCH_3$ | |
| 11.19 | H | H | H | H | H | $N-N(CH_3)_2$ | $COOCH_3$ | |
| 11.20 | $CH_3$ | $CH_3$ | H | H | H | $N-CH_3$ | $COOCH_3$ | |
| 11.21 | $CH_3$ | $CH_3$ | H | H | H | $N-NH_2$ | $COOCH_3$ | |
| 11.22 | $CH_3$ | $CH_3$ | H | H | H | $N-N(CH_3)_2$ | $COOCH_3$ | |

Tabelle 11 (Fortsetzung)

| Verb. Nr. | $R^1$ | $R^2$ | $R^3$ | $R^4$ | $X^1$ | Q | L | phys. Daten |
|---|---|---|---|---|---|---|---|---|
| 11.23 | $CH_3$ | H | H | H | H | $N-CH_3$ | $COOCH_3$ | |
| 11.24 | $CH_3$ | H | H | H | H | $N-N(CH_3)_2$ | $COOCH_3$ | |
| 11.25 | $CH_3$ | H | H | H | H | $N-NH_2$ | $COOCH_3$ | |
| 11.26 | H | H | H | H | H | $N-OCH_3$ | $COOCH_3$ | |
| 11.27 | H | H | H | H | H | $N-OC_2H_5$ | $COOCH_3$ | |
| 11.28 | H | H | H | H | H | $N-O-CH_2-CH=CH_2$ | $COOCH_3$ | Oel |
| 11.29 | H | H | H | H | H | $N-OC_4H_9-n$ | $COOCH_3$ | |
| 11.30 | H | H | H | H | H | $N-OC_6H_5$ | $COOCH_3$ | |
| 11.31 | $CH_3$ | $CH_3$ | H | H | H | $N-OCH_3$ | $COOCH_3$ | E-Form, Smp. 84-88°C |
| 11.32 | $CH_3$ | $CH_3$ | H | H | H | $N-OC_2H_5$ | $COOCH_3$ | Oel |
| 11.33 | $CH_3$ | $CH_3$ | H | H | H | N-OH | $COOCH_3$ | |
| 11.34 | $CH_3$ | $CH_3$ | H | H | H | $N-O-CH_2-CH=CH_2$ | $COOCH_3$ | |
| 11.35 | $CH_3$ | $CH_3$ | H | H | H | N-OH | $COOC_2H_5$ | |
| 11.36 | $CH_3$ | $CH_3$ | H | H | H | $N-OCH_3$ | $CONHCH_3$ | |
| 11.37 | $CH_3$ | $CH_3$ | H | H | H | $N-OCH_3$ | $COOC_4H_9-t$ | |
| 11.38 | $CH_3$ | H | H | H | H | $N-OCH_3$ | $COOCH_3$ | |
| 11.39 | $CH_3$ | H | H | H | H | N-OH | $COOCH_3$ | |
| 11.40 | $CH_3$ | H | H | H | H | $N-OC_2H_5$ | $COOCH_3$ | |
| 11.41 | $CH_3$ | H | H | H | H | $N-O-CH_2-CH=CH_2$ | $COOCH_3$ | |
| 11.42 | $CH_3$ | $CH_3$ | H | H | H | $N-O-C_6H_{13}-n$ | $COOCH_3$ | Oel |
| 11.43 | $CH_3$ | $CH_3$ | H | H | H | $N-O-CH_2-C_6H_5$ | $COOCH_3$ | Oel |
| 11.44 | $CH_3$ | $CH_3$ | H | H | H | $N-O-C_4H_9-n$ | $COOCH_3$ | Oel |
| 11.45 | $CH_3$ | $CH_3$ | H | H | H | $N-O-CH_2-CH=CHCl$ | $COOCH_3$ | Oel |
| 11.46 | $CH_3$ | $CH_3$ | H | H | H | $N-OCH_2-CH=CH_2$ | $COOCH_3$ | Oel |

Tabelle 12:

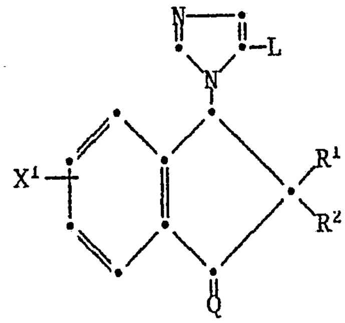

| Verb. Nr. | $R^1$ | $R^2$ | $X^1$ | Q | L | phys. Daten |
|---|---|---|---|---|---|---|
| 12.01 | $CH_3$ | $CH_3$ | H | $CH_2$ | $COOCH_3$ | Smp. 125-126°C |
| 12.02 | $CH_3$ | $CH_3$ | H | $C(CH_3)_2$ | $COOCH_3$ | |
| 12.03 | $CH_3$ | $CH_3$ | H | $C(C_6H_5)_2$ | $COOCH_3$ | |
| 12.04 | $CH_3$ | $CH_3$ | H | $CH-C_6H_5$ | $COOCH_3$ | |
| 12.05 | $CH_3$ | $CH_3$ | H | $CH_2$ | $COOC_2H_5$ | |
| 12.06 | $CH_3$ | H | H | $C(CH_3)_2$ | $COOCH_3$ | |
| 12.07 | $CH_3$ | H | H | $CH_2$ | $COOCH_3$ | |
| 12.08 | $CH_3$ | H | H | $CH-C_2H_5$ | $COOCH_3$ | |
| 12.09 | $CH_3$ | $CH_3$ | H | NH | $COOCH_3$ | |
| 12.10 | $CH_3$ | $CH_3$ | H | $N-NH_2$ | $COOCH_3$ | |
| 12.11 | $CH_3$ | $CH_3$ | H | $N-N(CH_3)_2$ | $COOCH_3$ | |
| 12.12 | $CH_3$ | H | H | $N-CH_3$ | $COOCH_3$ | |
| 12.13 | $CH_3$ | H | H | $N-N(CH_3)_2$ | $COOCH_3$ | |
| 12.14 | $CH_3$ | $CH_3$ | H | $N-OCH_3$ | $COOCH_3$ | Oel |
| 12.15 | $CH_3$ | $CH_3$ | H | N-OH | $COOCH_3$ | |
| 12.16 | $CH_3$ | $CH_3$ | H | $N-OC_2H_5$ | $COOCH_3$ | |
| 12.17 | $CH_3$ | $CH_3$ | H | $N-OC_6H_5$ | $COOCH_3$ | |
| 12.18 | $CH_3$ | $CH_3$ | H | $N-OCH_3$ | $COOC_2H_5$ | |
| 12.19 | $CH_3$ | H | H | $N-OCH_3$ | $COOCH_3$ | |
| 12.20 | $CH_3$ | H | H | N-OH | $COOCH_3$ | |
| 12.21 | $CH_3$ | H | H | $N-O-CH_2-CH=CH_2$ | $COOCH_3$ | |
| 12.22 | $CH_3$ | $CH_3$ | H | $N-C_6H_5$ | $COOCH_3$ | Harz |
| 12.23 | $CH_3$ | $CH_3$ | H | $N-C_6H_4-4-OCH_3$ | $COOCH_3$ | Harz |

Tabelle 13:

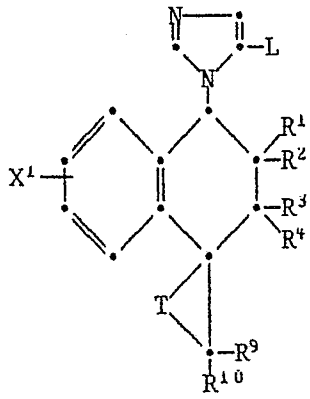

| Verb. Nr. | $R^1$ | $R^2$ | $R^3$ | $R^4$ | $X^1$ | $R^9$ | $R^{10}$ | T | L | phys. Daten |
|---|---|---|---|---|---|---|---|---|---|---|
| 13.01 | H | H | H | H | H | H | H | $CCl_2$ | $COOCH_3$ | |
| 13.02 | H | H | H | H | H | $CH_3$ | $CH_3$ | $CCl_2$ | $COOCH_3$ | |
| 13.03 | H | H | H | H | H | $C_2H_5$ | H | $CCl_2$ | $COOCH_3$ | |
| 13.04 | H | H | H | H | H | $C_6H_5$ | H | $CCl_2$ | $COOCH_3$ | |
| 13.05 | H | H | H | H | H | $C_2H_5$ | $C_2H_5$ | $CCl_2$ | $COOCH_3$ | |
| 13.06 | $CH_3$ | $CH_3$ | H | H | H | $CH_3$ | $CH_3$ | $CCl_2$ | $COOCH_3$ | |
| 13.07 | $CH_3$ | $CH_3$ | H | H | H | H | H | $CCl_2$ | $COOCH_3$ | |
| 13.08 | $CH_3$ | $CH_3$ | H | H | H | $C_2H_5$ | H | $CCl_2$ | $COOCH_3$ | |
| 13.09 | $CH_3$ | $CH_3$ | H | H | H | $C_6H_5$ | $C_6H_5$ | $CCl_2$ | $COOCH_3$ | |
| 13.10 | $CH_3$ | $CH_3$ | H | H | H | H | H | $CCl_2$ | $COOC_2H_5$ | |
| 13.11 | $CH_3$ | $CH_3$ | H | H | H | H | H | $CCl_2$ | $COOC_4H_9$-t | |
| 13.12 | $CH_3$ | H | H | H | H | $CH_3$ | $CH_3$ | $CCl_2$ | $COOCH_3$ | |
| 13.13 | $CH_3$ | H | H | H | H | H | H | $CCl_2$ | $COOCH_3$ | |
| 13.14 | $CH_3$ | H | H | H | H | $C_6H_5$ | $C_6H_5$ | $CCl_2$ | $COOCH_3$ | |
| 13.15 | $CH_3$ | H | H | H | H | $C_6H_5$ | H | $CCl_2$ | $COOCH_3$ | |
| 13.16 | $CH_3$ | $CH_3$ | H | H | H | $CH_3$ | $CH_3$ | O | $COOCH_3$ | |
| 13.17 | H | H | H | H | H | H | H | O | $COOCH_3$ | |
| 13.18 | H | H | H | H | H | $CH_3$ | $CH_3$ | O | $COOCH_3$ | |
| 13.19 | H | H | H | H | H | $C_2H_5$ | H | O | $COOCH_3$ | |
| 13.20 | H | H | H | H | H | $C_6H_5$ | H | O | $COOCH_3$ | |
| 13.21 | H | H | H | H | H | $C_2H_5$ | $C_2H_5$ | O | $COOCH_3$ | |
| 13.22 | $CH_3$ | $CH_3$ | H | H | H | H | H | O | $COOCH_3$ | |
| 13.23 | $CH_3$ | $CH_3$ | H | H | H | $C_2H_5$ | H | O | $COOCH_3$ | |

Tabelle 13 (Fortsetzung)

| Verb. Nr. | $R^1$ | $R^2$ | $R^3$ | $R^4$ | $X^1$ | $R^9$ | $R^{10}$ | T | L | phys. Daten |
|---|---|---|---|---|---|---|---|---|---|---|
| 13.24 | $CH_3$ | $CH_3$ | H | H | H | $C_6H_5$ | $C_6H_5$ | O | $COOCH_3$ | |
| 13.25 | $CH_3$ | $CH_3$ | H | H | H | H | H | O | $COOC_2H_5$ | |
| 13.26 | $CH_3$ | $CH_3$ | H | H | H | H | H | O | $CONHCH_3$ | |
| 13.27 | $CH_3$ | $CH_3$ | H | H | H | H | H | O | $COOC_4H_9$-t | |
| 13.28 | $CH_3$ | H | H | H | H | $CH_3$ | $CH_3$ | O | $COOCH_3$ | |
| 13.29 | $CH_3$ | H | H | H | H | H | H | O | $COOCH_3$ | |
| 13.30 | $CH_3$ | H | H | H | H | $C_6H_5$ | $C_6H_5$ | O | $COOCH_3$ | |
| 13.31 | $CH_3$ | H | H | H | H | $C_6H_5$ | H | O | $COOCH_3$ | |

Tabelle 14:

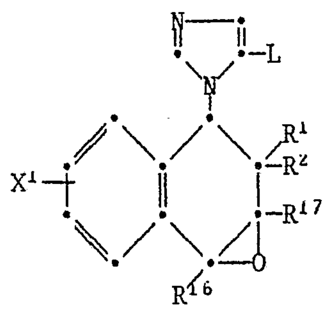

| Verb. Nr. | $R^1$ | $R^2$ | $X^1$ | $R^{16}$ | $R^{17}$ | L | phys. Daten |
|---|---|---|---|---|---|---|---|
| 14.01 | H | H | H | H | H | $COOCH_3$ | |
| 14.02 | $C_2H_5$ | $C_2H_5$ | H | H | $CH_3$ | $COOCH_3$ | |
| 14.03 | $CH_3$ | H | H | H | H | $COOCH_3$ | |
| 14.04 | $CH_3$ | H | H | H | H | $COOC_2H_5$ | Smp. 60-68°C |
| 14.05 | $CH_3$ | $CH_3$ | H | H | H | $COOCH_3$ | |
| 14.06 | $CH_3$ | $CH_3$ | H | H | H | $COOC_2H_5$ | |
| 14.07 | $CH_3$ | $CH_3$ | H | H | H | $COOC_4H_9$-t | |
| 14.08 | $CH_3$ | $CH_3$ | H | H | H | $CONHCH_3$ | |
| 14.09 | $CH_3$ | $CH_3$ | H | H | H | $CONH-OCH_3$ | |
| 14.10 | $CH_3$ | $CH_3$ | H | H | $CH_3$ | $COOCH_3$ | |

Tabelle 15:
(Zwischenprodukte)

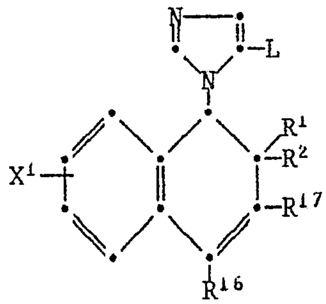

| Verb. Nr. | $R^1$ | $R^2$ | $X^1$ | $R^{16}$ | $R^{17}$ | L | phys. Daten |
|---|---|---|---|---|---|---|---|
| 15.01 | H | H | H | H | H | $COOCH_3$ | |
| 15.02 | $C_2H_5$ | $C_2H_5$ | H | H | $CH_3$ | $COOCH_3$ | |
| 15.03 | $CH_3$ | H | H | H | H | $COOCH_3$ | |
| 15.04 | $CH_3$ | H | H | H | H | $COOC_2H_5$ | |
| 15.05 | $CH_3$ | $CH_3$ | H | H | H | $COOCH_3$ | Smp. 60-62°C |
| 15.06 | $CH_3$ | $CH_3$ | H | H | H | $COOC_2H_5$ | |
| 15.07 | $CH_3$ | $CH_3$ | H | H | H | $COOC_4H_9$-t | |
| 15.08 | $CH_3$ | $CH_3$ | H | H | H | $CONHCH_3$ | |
| 15.09 | $CH_3$ | $CH_3$ | H | H | H | $CONH-OCH_3$ | |
| 15.10 | $CH_3$ | $CH_3$ | H | H | $CH_3$ | $COOCH_3$ | |

Formulierungsbeispiele

Beispiel F1: Formulierungsbeispiele für flüssige Wirkstoffe der Formel I

(% = Gewichtsprozent)

| a) Emulsionskonzentrate | a) | b) | c) |
|---|---|---|---|
| Wirkstoff Nr. 5.07 | 20 % | 40 % | 50 % |
| Ca-Dodecylbenzolsulfonat | 5 % | 8 % | 5,8 % |
| Ricinusöl-polyäthylenglykoläther (36 Mol AeO) | 5 % | - | - |
| Tributylphenol -polyäthylenglykoläther (30 mol AeO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) Lösungen | a) | b) | c) | d) |
|---|---|---|---|---|
| Wirkstoff Nr. 5.07 | 80 % | 10 % | 5 % | 95 % |
| Aethylenglykol-monomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösung sind zur Anwendung in Form kleinster Tropfen geeignet.

| c) Granulate | a) | b) |
|---|---|---|
| Wirkstoff Nr. 5.06 | 5 % | 10 % |
| Kaolin | 94 % | - |
| Hochdisperse Kieselsäure | 1 % | |
| Attapulgit | - | 90 % |

Der Wirkstoff wird in Methylenchlorid gelöst, auf den Träger aufgesprüht und das Lösungsmittel anschliessend im Vakuum abgedampft.

| d) Stäubemittel | a) | b) |
|---|---|---|
| Wirkstoff Nr. 5.06 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertiges Stäubemittel.

Beispiel F2: Formulierungsbeispiele für feste Wirkstoffe der Formel I

(% = Gewichtsprozent)

| a) Spritzpulver | a) | b) |
|---|---|---|
| Wirkstoff Nr. 1.05 | 20 % | 60 % |
| Na-Ligninsulfonat | 5 % | 5 % |
| Na-Laurylsulfat | 3 % | - |
| Na-Diisobutylnaphthalinsulfonat | - | 6 % |
| Octylphenolpolyäthylenglykoläther (7-8 Mol AeO) | - | 2 % |
| Hochdisperse Kieselsäure | 5 % | 27 % |
| Kaolin | 67 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsions-Konzentrat

| | |
|---|---|
| Wirkstoff Nr. 1.05 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff Nr. 2.01 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

d) Extruder Granulat
Wirkstoff Nr. 1.21 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat
Wirkstoff Nr. 1.21 3 %
Polyäthylenglykol (MG 200) 3 %
Kaolin 94 %

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat
Wirkstoff Nr. 3.05 40 %
Aethylenglykol 10 %
Nonylphenolpolyäthylenglykoläther (15 Mol AeO) 6 %
Na-Ligninsulfonat 10 %
Carboxymethylcellulose 1 %
37%ige wässrige Formaldehyd-Lösung 0,2 %
Silikonöl in Form einer 75%igen wässrigen Emulsion 0,8 %
Wasser 32 %

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel B1: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25%igen Emulsionskonzentrat, behandelt. Es werden Konzentrationen von 4 kg Wirksubstanz/Hektar angewendet. Die Saatschalen werden im Gewächshaus bei 22-25° C und 50-70 % relativer Luftfeuchtigkeit gehalten und der Versuch nach 3 Wochen ausgewertet.

1 : Pflanze nicht gekeimt oder total abgestorben
2-3: sehr starke Wirkung
4-6: mittlere Wirkung
7-8: schwache Wirkung
9: keine Wirkung (wie unbehandelte Kontrolle).

In diesem Versuch zeigen die Verbindungen der Tabellen 1 bis 14 starke Herbizidwirkung.

Testresultate: preemergente Prüfung

Aufwandmenge: 4 kg Aktivsubstanz pro Hektar.

| Verb. Nr. | Avena | Setaria | Sinapis | Stellaria |
|---|---|---|---|---|
| 1.01 | 3 | 2 | 3 | 3 |
| 1.05 | 2 | 1 | 2 | 2 |
| 1.21 | 1 | 1 | 2 | 2 |
| 1.22 | 2 | 1 | 2 | 4 |
| 2.01 | 2 | 1 | 2 | 2 |
| 3.01 | 4 | 2 | 2 | 3 |
| 3.05 | 2 | 1 | 2 | 2 |
| 3.21 | 2 | 1 | 2 | 2 |
| 4.01 | 2 | 1 | 2 | 2 |
| 4.12 | 2 | 1 | 2 | 2 |
| 5.06 | 2 | 1 | 2 | 2 |
| 5.07 | 2 | 1 | 2 | 2 |
| 7.07 | 4 | 2 | 3 | 1 |
| 8.02 | 3 | 1 | 2 | 3 |
| 11.31 | 3 | 1 | 2 | 1 |
| 11.32 | 3 | 2 | 1 | 1 |
| 11.33 | 2 | 3 | 2 | 2 |
| 12.01 | 5 | 1 | 2 | 2 |
| 12.14 | 3 | 2 | 1 | 1 |

Beispiel B2: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°-26°C und 45-60° % relativer Luftfeuchtigkeit gehalten. 15 Tage nach der Behandlung wird der Versuch nach der im preemergenten Versuch angegebenen Notenskala ausgewertet.

Auch in diesem Versuch zeigen die Verbindungen der Tabelle 1 bis 14 gute Herbizidwirkung.

Testresultate: postemergente Prüfung

Aufwandmenge: 4 kg Aktivsubstanz pro Hektar.

| Verb. Nr. | Avena | Setaria | Lolium | Solanum | Sinapis | Stellaria | Phaseolus |
|---|---|---|---|---|---|---|---|
| 1.01 | 3 | 4 | 4 | 3 | 2 | 4 | 2 |
| 1.21 | 4 | 4 | 3 | 3 | 3 | 3 | 2 |
| 1.22 | 3 | 4 | 4 | 3 | 3 | 2 | 2 |
| 2.01 | 4 | 4 | 3 | 2 | 3 | 4 | 2 |
| 4.01 | 4 | 4 | 4 | 3 | 4 | 5 | 2 |
| 4.12 | 4 | 3 | 4 | 2 | 3 | 4 | 2 |
| 5.06 | 4 | 4 | 4 | 4 | 3 | 4 | 2 |
| 5.07 | 4 | 4 | 4 | 2 | 3 | 4 | 2 |
| 11.31 | 7 | 4 | 6 | 3 | 2 | 3 | 2 |
| 11.32 | 6 | 5 | 4 | 4 | 2 | 3 | 2 |
| 12.01 | 5 | 4 | 4 | 2 | 3 | 3 | 2 |
| 12.14 | 6 | 5 | 4 | 4 | 2 | 3 | 2 |

Beispiel B3 Herbizidwirkung bei Wasserreis (paddy)

Die Wasserunkräuter Echinochloa crus galli und Monochoria vaginalis werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe. Die dabei verwendete Dosis entspricht einer Wirkstoffmenge von 4 kg AS pro Hektar (Spritzbrühmenge = 550 1/ha.) Die bepflanzten Becher werden dann im Gewächshaus unter optimalen Wachstumsbedingungen für die Reisunkräuter aufgestellt, d.h. bei 25°-30°C und hoher Luftfeuchtigkeit. Die Auswertung der Versuche findet 3 Wochen nach Applikation nach dem gleichen Massstab wie im preemergenten Versuch B1 statt. Die Verbindungen der Tabellen 1 bis 14 schädigen dabei die Unkräuter, nicht aber den Reis.

Testresultate: Wasserunkräuter

Aufwandmenge: 4 kg Aktivsubstanz pro Hektar.

| Verb. Nr. | Echinochloa | Monochoria |
|---|---|---|
| 1.01 | 1 | 1 |
| 1.05 | 1 | 1 |
| 1.21 | 1 | 1 |
| 1.22 | 1 | 1 |
| 1.23 | 2 | 1 |
| 2.01 | 1 | 1 |
| 3.01 | 1 | 1 |
| 3.05 | 1 | 1 |
| 3.21 | 1 | 1 |
| 3.22 | 1 | 1 |
| 4.01 | 1 | 1 |
| 4.12 | 1 | 1 |
| 5.06 | 1 | 1 |
| 5.07 | 1 | 1 |
| 7.07 | 1 | 1 |
| 8.02 | 1 | 1 |
| 11.31 | 1 | 1 |
| 11.32 | 1 | 1 |
| 11.33 | 1 | 1 |
| 11.42 | 1 | 1 |
| 11.43 | 1 | 1 |
| 11.44 | 1 | 1 |
| 11.45 | 1 | 1 |
| 11.46 | 1 | 1 |
| 12.01 | 1 | 1 |
| 12.14 | 1 | 1 |

## Patentansprüche

1. 1,5-substituierte Imidazolderivate der Formel I

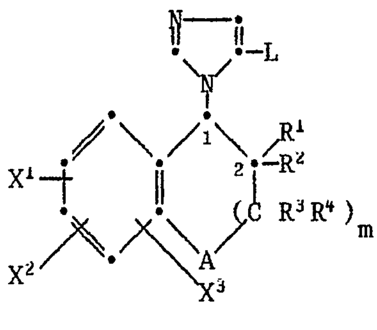

(I),

die stereochemisch isomeren Formen davon, sowie deren Salze, worin m die Zahl null oder eines, A eine Gruppe

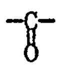 ,

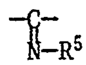 ,

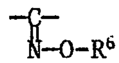 ,

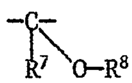 ,

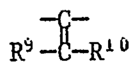 ,

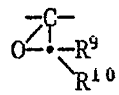 ,

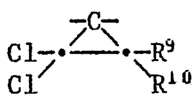 ,

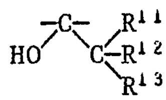 oder

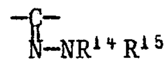

oder falls m für die Zahl null steht auch eine Gruppe

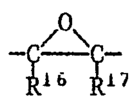 ,

L Cyano oder eine Gruppe

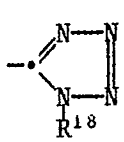 , 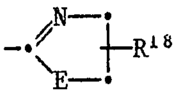 , 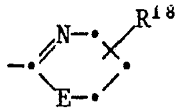 , 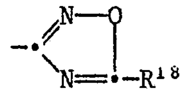 ,

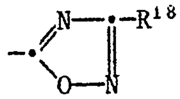 , 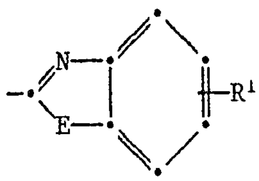 , 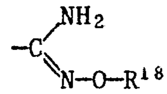

oder 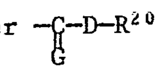 ,

$X^1$ Wasserstoff, $C_1$-$C_5$-Alkyl, $C_1$-$C_5$-Alkoxy, Halogen, $C_2$-$C_5$-Alkenyl, Nitro, Amino, $C_1$-$C_5$-Alkylcarbonylamino, Trifluormethyl oder Difluormethoxy,
$X^2$ Wasserstoff, $C_1$-$C_5$-Alkyl, Halogen oder $C_1$-$C_5$-Alkoxy,
$X^3$ Wasserstoff oder Halogen,
$R^1$ und $R^2$ unabhängig voneinander Wasserstoff, $C_1$-$C_5$-Alkyl oder $C_2$-$C_5$-Alkenyl, oder
$R^1$ und $R^2$ zusammen mit dem sie tragenden Kohlenstoffatom einen spirocyclischen $C_3$-$C_7$-Cycloalkanring,
$R^3$ Wasserstoff, $C_1$-$C_5$-Alkyl, Halogen, $C_1$-$C_5$-Alkoxy oder Hydroxy,
$R^4$ Wasserstoff, $C_1$-$C_5$-Alkyl, Halogen oder $C_1$-$C_5$-Alkoxy, oder
$R^3$ und $R^4$ zusammen mit dem sie tragenden Kohlenstoffatom eine Carbonylgruppe, oder
$R^2$ und $R^3$ eine $C_2$-$C_5$-Alkylenbrücke bedeuten, wobei
$R^5$, $R^6$, $R^7$, $R^9$ und $R^{16}$ jeweils für Wasserstoff, $C_1$-$C_5$-Alkyl, -$CH_2$-$C_2$-$C_5$-Alkenyl, Benzyl oder Phenyl,
$R^8$ für Wasserstoff, -CO-$R^{21}$ oder -$SO_2$-$R^{22}$
$R^{10}$, $R^{11}$, $R^{12}$ und $R^{17}$ jeweils unabhängig voneinander für Wasserstoff, $C_1$-$C_5$-Alkyl, $C_2$-$C_5$-Alkenyl oder Phenyl,
$R^{13}$ für Wasserstoff, Halogen, $C_1$-$C_5$-Alkoxy, Amino, $C_1$-$C_5$-Alkylamino oder Di-$C_1$-$C_5$-Alkylamino
$R^{14}$ und $R^{15}$ unabhängig voneinander für Wasserstoff oder $C_1$-$C_5$-Alkyl,
E für Sauerstoff, Schwefel oder -$NR^{18}$-,
$R^{18}$ für Wasserstoff oder $C_1$-$C_5$-Alkyl,
$R^{19}$ für Wasserstoff, $C_1$-$C_5$-Alkyl, $C_1$-$C_5$-Alkoxy, Halogen, Nitro, Trifluormethyl oder Difluormethoxy,
G für Sauerstoff, Schwefel oder =N-$R^{20}$,
D für Sauerstoff, Schwefel, -$NR^{23}$-,
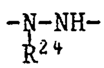 oder 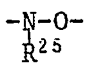
$R^{20}$, $R^{23}$, $R^{24}$ und $R^{25}$ unabhängig voneinander für Wasserstoff, $C_1$-$C_5$-Alkyl, -$CH_2$-$C_2$-$C_5$-Alkenyl, -$CH_2$-$C_2$-$C_6$-Alkinyl, $C_3$-$C_7$-Cycloalkyl oder mit $C_3$-$C_7$-Cycloalkyl, $C_3$-$C_7$-Cycloalkoxy, Phenyl, $C_1$-$C_5$-Alkoxy, Hydroxy, Cyan,

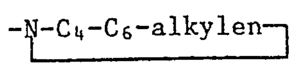 ,

Carboxyl oder $C_1$-$C_5$-Alkoxycarbonyl substituiertes $C_1$-$C_5$-Alkyl, oder
$R^{20}$ und $R^{23}$ zusammen mit den sie tragenden Stickstoffatom für einen gegebenenfalls durch $C_1$-$C_5$-Alkyl substituierten Piperidinyl-, Pyrrolidinyl-, Morpholinyl- oder Thiomorpholinylring,
$R^{21}$ für gegebenenfalls durch $C_1$-$C_5$-Alkoxy oder 1 bis 3 Halogenatome substituiertes $C_2$-$C_5$-Alkyl oder für gegebenenfalls durch 1 bis 2 Substituenten aus der Gruppe $C_1$-$C_5$-Alkyl, $C_1$-$C_5$-Alkoxy, Halogen, $C_1$-$C_5$-Halogenalkyl, $C_1$-$C_5$-Halogenalkoxy, Nitro oder Benzyl substituiertes Phenyl, und
$R^{22}$ für $C_1$-$C_5$-Alkyl, $C_1$-$C_5$-Halogenalkyl oder gegebenenfalls durch 1 bis 2 Substituenten aus der Gruppe $C_1$-$C_5$-Alkyl, $C_1$-$C_5$-Alkoxy, $C_1$-$C_5$-Halogenalkyl, $C_1$-$C_5$-Halogenalkoxy, Halogen oder Nitro substituiertes Phenyl stehen.

2. Verbindungen gemäss Anspruch 1, dadurch gekennzeichnet, dass $X^2$ und $X^3$ Wasserstoff bedeuten.

3. Verbindungen gemäss Anspruch 2, dadurch gekennzeichnet, dass $X^1$ für Wasserstoff, Chlor, Methyl oder Methoxy steht.

4. Verbindungen gemäss Anspruch 1, dadurch gekennzeichnet, dass L für -$COOR^{20}$ oder -$CONR^{20}R^{23}$ steht.

5. Verbindungen gemäss Anspruch 4, dadurch gekennzeichnet, dass L für Carboxyl, Carbamoyl,

$C_1$-$C_5$-Alkoxycarbonyl, $C_1$-$C_5$-Alkylcarbamoyl, Di-$C_1$-$C_5$-Alkylcarbamoyl oder $C_1$-$C_5$-Alkoxycarbamoyl steht.

6. Verbindungen gemäss Anspruch 5, dadurch gekennzeichnet, dass L für Methoxycarbonyl, Aethoxycarbonyl, Methylcarbamoyl oder Methoxycarbamoyl steht.

7. Verbindungen gemäss Anspruch 1, dadurch gekennzeichnet, dass $R^1$, $R^2$, $R^3$ und $R^4$ unabhängig voneinander für Wasserstoff oder Methyl stehen.

8. Verbindungen gemäss Anspruch 1, dadurch gekennzeichnet, dass A für die Gruppe

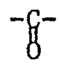 , -CHOH- , 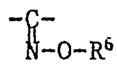 , 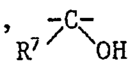 , 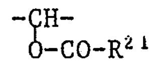 , 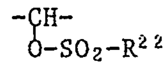 oder

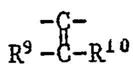

steht, worin $R^6$, $R^7$, $R^9$ und $R^{10}$ unabhängig voneinander jeweils für Wasserstoff, $C_1$-$C_5$-Alkyl oder Phenyl, $R^{21}$ für $C_1$-$C_5$-Alkyl, $C_1$-$C_5$-Chloralkyl, $C_2$-$C_4$-Alkoxyalkyl oder Phenyl und $R^{22}$ für $C_1$-$C_5$-Alkyl oder gegebenenfalls durch Methyl, Chlor oder Nitro substituiertes Phenyl stehen.

9. Verbindungen gemäss Anspruch 8, dadurch gekennzeichnet, dass A für die Gruppe

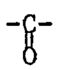 , -CHOH , 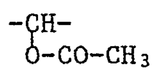 , 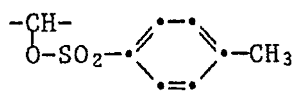 , 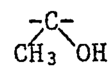 , .

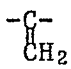 oder 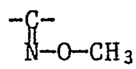 steht.

10. Verbindungen gemäss Anspruch 1, dadurch gekennzeichnet, dass $X^1$ Wasserstoff, Chlor, Methyl oder Methoxy, $X^2$ Wasserstoff, Methyl oder Methoxy, $X^3$ Wasserstoff, L die Gruppe $-COOR^{20}$ oder $-CONR^{20}R^{23}$, $R^1$, $R^2$, $R^3$ und $R^4$ unabhängig voneinander Wasserstoff oder Methyl und A die Gruppe

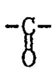 , -CHOH- , 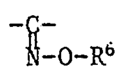 , 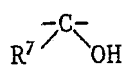 , 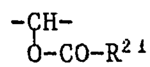 , 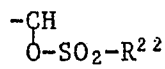

oder

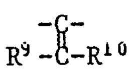

bedeuten, worin $R^6$, $R^7$, $R^9$ und $R^{10}$ unabhängig voneinander jeweils für Wasserstoff, $C_1$-$C_5$-Alkyl oder Phenyl, $R^{21}$ für $C_1$-$C_5$-Alkyl, $C_1$-$C_5$-Chloralkyl, $C_2$-$C_4$-Alkoxyalkyl oder Phenyl und $R^{22}$ für $C_1$-$C_5$-Alkyl oder gegebenenfalls durch Methyl, Chlor oder Nitro substituiertes Phenyl stehen.

11. Verbindungen gemäss Anspruch 10, dadurch gekennzeichnet, dass L für Carboxyl, Carbamoyl, $C_1$-$C_5$-Alkoxycarbonyl, $C_1$-$C_5$-Alkylcarbamoyl, Di-$C_1$-$C_5$-Alkylcarbamoyl oder $C_1$-$C_5$-Alkoxycarbamoyl und A für die Gruppe

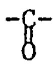 , -CHOH- , 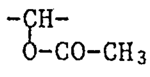 , 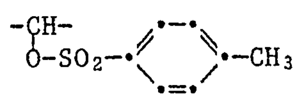 , 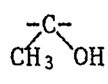 ,

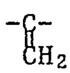 oder 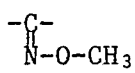 stehen.

12. Verbindungen gemäss Anspruch 11, dadurch gekennzeichnet, dass L für Methoxycarbonyl, Aethoxycarbonyl, Methylcarbamoyl oder Methoxycarbamoyl steht.
13. 1-(2,2-Dimethyl-3-oxo-indan-1-yl)-5-imidazol-carbonsäure-methylester, gemäss Anspruch 1.
14. 1-(2,2-Dimethyl-4-oxo-tetralin-1-yl)-5-imidazol-carbonsäure-methylester, gemäss Anspruch 1.
15. 1,3-trans-1-(3-Brom-2,2-dimethyl-4-oxo-tetralin-1-yl)-5-imidazolcarbonsäure-methylester, gemäss Anspruch 1.
16. 1,3-cis-1-(3-Brom-2,2-dimethyl-4-oxo-tetralin-1-yl)-5-imidazolcarbonsäure-methylester, gemäss Anspruch 1.
17. 1,4-trans-1-(2,2-Dimethyl-4-hydroxy-tetralin-1-yl)-5-imidazolcarbonsäure-methylester, gemäss Anspruch 1.
18. 1,3-cis-1-(2,2-Dimethyl-3-hydroxy-indan-1-yl)-5-imidazolcarbonsäure-methylester, gemäss Anspruch 1.
19. 1,3-trans-1-(2,2-Dimethyl-3-hydroxy-indan-1-yl)-5-imidazolcarbonsäure-methylester, gemäss Anspruch 1.
20. 1,4-cis-1-(4-Acetoxy-2,2-dimethyl-tetralin-1-yl)-5-imidazolcarbonsäure-methylester, gemäss Anspruch 1.
21. 1,4-trans-1-(4-Acetoxy-2,2-dimethyl-tetralin-1-yl)-5-imidazolcarbonsäure-methylester, gemäss Anspruch 1,
22. 1-(2,2-Dimethyl-3-methylidenyl-indan-1-yl)-5-imidazolcarbonsäure-methylester, gemäss Anspruch 1.
23. Verfahren zur Herstellung der Verbindungen an der Formel I gemäss Anspruch 1, dadurch gekennzeichnet, dass man eine Verbindung der Formel II

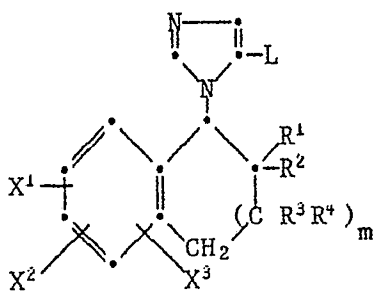

(II)

worin L für Cyan oder $C_1$-$C_5$-Alkoxycarbonyl steht und $R^1$, $R^2$, $R^3$, $R^4$, $X^1$, $X^2$, $X^3$ und m die unter Formel I gegebenen Bedeutungen haben, mit einem Oxidationsmittel oxidiert und gewünschtenfalls durch Derivatisierung der entstehenden Carbonylgruppe und/oder des Substituenten L in die übrigen Verbindungen der Formel I überführt.
24. Herbizides Mittel, dadurch gekennzeichnet, dass es neben Träger-und/oder anderen Zuschlagstoffen als Wirkstoff mindestens ein 1,5-substituiertes Imidazol-Derivat der Formel I, gemäss Anspruch 1 enthält.
25. Verfahren zur Bekämpfung von Unkräutern, dadurch gekennzeichnet, dass man die Unkräuter oder ihren Lebensraum mit einer herbizid wirksamen Menge einer Verbindung der Formel I, gemäss Anspruch 1, behandelt.
26. Verfahren gemäss Anspruch 25 zur selektiven Bekämpfung von Unkräutern in Nutzpflanzenkulturen.
27. Verfahren gemäss Anspruch 26, dadurch gekennzeichnet, dass es sich bei den Nutzpflanzenkulturen um Getreide, Zuckerrüben, Raps, Sojabohnen, Mais oder Reis handelt.
28. Verfahren gemäss Anspruch 26, dadurch gekennzeichnet, dass es sich bei der Nutzpflanzenkultur um Reis handelt.
29. Verfahren gemäss Anspruch 26, dadurch gekennzeichnet, dass es sich bei der Nutzpflanzenkultur um Mais handelt.

Europäisches Patentamt

# EUROPÄISCHER RECHERCHENBERICHT

Nummer der Anmeldung

EP 88810559.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP - A2/A3 - 0 207 563 (JANSSEN PHARMACEUTICA N.V.)<br>* Ansprüche 1,15,21,26 *<br>-- | 1,23-29 | C 07 D 233/90<br>C 07 D 405/10<br>A 01 N 43/50 |
| A | CHEMICAL ABSTRACTS, Band 67, Nr. 25, 18. Dezember 1967, Columbus, Ohio, USA<br>VANBREUSEGHEM, R.; CUTSEM VAN, J.; THIENPONT, D. "Experimental study of a new fungistatic agent: R 10100 (1-(1,2,3,4-tetrahydro-1--naphthyl)-5-(ethoxycarbonyl)--imidazole nitrate)"<br>Seite 10880, Spalte 2, Zusammenfassung-Nr. 115 596z<br>& Chemotherapia 12(2), 107-22 (1967)<br>-- | 1,23 | |
| A | CHEMICAL ABSTRACTS, Band 68, Nr. 21, 20. Mai 1968, Columbus, Ohio, USA<br>GODEFROI; E.F.; VAN CUTSEM, J.; VAN DER EYCKEN, C.A.M., JANSSEN, P.A.J. "1-(1-Indanyl)- and 1-(1--tetralyl)imidazole-5-carboxylete esters, a novel type of antifungel agents"<br>Seite 9239, Spalte 1, Zusammenfassung-Nr. 95 758h<br>& J. Med. Chem. 10(6), 1160-1 (1967)<br>-- | 1,23 | RECHERCHIERTE SACHGEBIETE (Int. Cl.4)<br>C 07 D 233/00<br>C 07 D 405/00 |

Der vorliegende Recherchenbericht wurde für alle Patentansprüche erstellt.

| Recherchenort | Abschlußdatum der Recherche | Prüfer |
|---|---|---|
| WIEN | 16-11-1988 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

# EUROPÄISCHER RECHERCHENBERICHT

Nummer der Anmeldung

EP 88810559.0



## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 69, Nr. 5, 29. Juli 1968, Columbus, Ohio, USA<br>JANSSEN PHARMACEUTICA N.V. "Imidazole-carboxylic acid esters"<br>Seite 1807, Spalte 2, Zusammenfassung-Nr. 19 160y<br>& Neth. Appl. 67 05 095<br>---- | 1,23-29 | |

RECHERCHIERTE SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| WIEN | 16-11-1988 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82